(19)

Europäisches
Patentamt

European
Patent Office

Office européen
des brevets

(11) **EP 3 354 269 A1**

(12) **EUROPEAN PATENT APPLICATION**

(43) Date of publication:
**01.08.2018 Bulletin 2018/31**

(21) Application number: **18153496.7**

(22) Date of filing: **25.01.2018**

(51) Int Cl.:
*A61K 31/765* (2006.01)    *A61K 8/86* (2006.01)
*A61K 36/00* (2006.01)    *A23L 29/206* (2016.01)
*A23L 33/00* (2016.01)    *A61P 3/06* (2006.01)
*A61P 3/04* (2006.01)    *A61P 3/08* (2006.01)
*A61P 3/10* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **26.01.2017 CZ 20170040**

(71) Applicants:
• **Vidia spol. s r.o.**
**25242 Jesenice u Prahy - Vestec (CZ)**
• **Fyziologicky Ustav AV CR, v.v.i.**
**142 20 Praha (CZ)**

(72) Inventors:
• **Labaj, Juraj**
**12800 Praha 2 - Nusle (CZ)**
• **Polakova, Michaela**
**14200 Praha 4 (CZ)**
• **Rossmeisl, Martin**
**10700 Praha 112 - Dubec (CZ)**
• **Kopecky, Jan**
**18200 Praha 8 (CZ)**
• **Pavlisova, Jana**
**25162 Mukarov (CZ)**

(74) Representative: **Hartvichova, Katerina**
**Harber IP s.r.o.**
**Na belidle 64/3**
**150 00 Praha 5 (CZ)**

(54) **USE OF PREPARATION FOR ORAL ADMINISTRATION CONTAINING LIGNIN**

(57)    The present invention relates to a preparation for oral administration containing lignin for use in the prevention and/or treatment of obesity and/or obesity-induced metabolic complications (abdominal fat accumulation, glucose intolerance, hepatic steatosis) in humans suffering from obesity. It further relates to the use of the preparation containing lignin as a food supplement to reduce overweight and/or to improve the overweight-induced metabolic complications in overweight individuals.

EP 3 354 269 A1

**Description**

Field of Art

The present invention relates to the use of a preparation for oral administration comprising lignin as a medicament and/or as a food supplement.

Background Art

Lignin, which, together with cellulose, forms the main components of the cell wall of plants, is one of the most widespread organic substances in nature. Lignin molecules are an amorphous, highly branched aromatic biopolymer formed in the plant biosynthesis process by radical polymerization of the p-coumaryl, coniferyl and sinapyl alcohols to form phenylpropane units, for example guaiacyl and syringyl units with hydroxyl or carbonyl substitutions (Higuchi, T.: Lignin structure and morphological distribution in plant cell walls. In: Lignin biodegradation: Microbiology, Chemistry, and Potential Application. Kirk, V.I., Higuchi, T., Chang, H. (eds). Florida: CRC Press, 1-7, 1980). The presence of lignin in plant tissue represents protection against mechanical, biochemical and environmental stress. Lignin inter alia reduces water sorption, thereby increasing spatial stability of plant tissue and inhibiting the biological effect of fungi on wood. From a chemical point of view, it acts like an adhesive, antioxidant and complex-forming compound.
Lignin is also quantitatively the most significant by-product of the paper industry (Glasser, W.G.; Forest Products Journal 31, 1981, p. 24-29). Although waste lignin is a potentially rich renewable resource whose accumulation also has a negative impact on the environment, its practical use is often limited to combustion in the process of recovering cooking chemicals in pulp production. The technologies are already known which use waste to optimize delignification by replacement of anthraquinone derivatives in alkaline delignification processes, or for dyeing polyester fibres in textile industry.

Current research also focuses on exploitation of chemically modified lignin isolates, whose structure as well as chemical and physical properties depend on the process of isolation and chemical modification of paper waste. Thus chemically modified lignin can be used for example for the production of phenol formaldehyde resins, polyurethane foams or biodegradable polymeric surfactants - diversants. In addition, lignin is able to modify the surface properties of polypropylene matrix, which then becomes biodegradable by ligninolytic micromycetes. The study of antimicrobial effects of lignin preparations further showed an inhibitory effect on the growth of pathogenic yeasts, indicating its potential application in agriculture or in fermentation industry.

Recently, the number of publications has increased that point to bioactive effects of lignins and their possible use in medicine. In this respect, anti-carcinogenic and anti-mutational effects of both synthetic and natural lignin products are particularly important as well as their inhibitory effects on the growth of HIV and influenza A viruses. Furthermore, it has been demonstrated that different lignin preparations, unlike cellulose and hemicellulose, show significant adsorption properties for N-nitrosamines and bile acids, wherein this binding capacity may be further enhanced by chemical and biological modifications, for example biotransformations of polymeric lignin by yeast (*Sporobolomyces roseus* and *Geotrichum klebahuii*). Due to the presence of hydroxyl groups which are preferably shielded with methoxy groups, lignins may also exhibit antioxidant activity and function as stabilizers of oxygen radical and radical induced by reduction reactions (Kratzl, K., Schaefer, W., Claus, P., Gratz, J., Schilling, P.; Monatsh. Chem. 98, 891-904, 1964; Lu FJ, Chu LH, Gau RJ.; Nutr Cancer 30(1):31-8, 1998). Adsorption and antioxidant activity of lignins isolated from beechwood hydrolysates were confirmed in both *in vitro* and *ex vivo* conditions.

As already mentionned, the properties of lignin and possibilities of its use depend on the origin and source of lignin itself, the isolation process and its subsequent modification. The process of isolation of lignin as disclosed in patent no. SK28259 begins by neutralizing the leachate (a mixture of lignin-rich substances). This solution is then heated to coagulate lignin and accelerate subsequent filtration. The precipitation process is followed by filtration of lignin, washing with water, drying and yield determination. The next step is extraction of lignin with 0.1M sodium hydroxide, which is again followed by filtration, washing with water, drying and yield determination. Fractionation of thus modified lignin, and filtration of the insoluble component, results in the isolation of soluble lignin fraction. After evaporation of the solvents, drying and precipitating, the prepared fraction is subsequently repeatedly extracted with 0.1M sodium hydroxide to form an alkaline solution of lignin which is further processed.

The initial raw material for isolation of lignin, as disclosed in document SK 280859, is a mixture of lignin-rich substances which is produced as a by-product in the manufacture of pulp. The technological process leads to preparation of a biologically active lignin product that distinguishes from other commercially available lignins by a unique molecular structure, specifically by presence of polymerized polyphenol building blocks that provide for the optimal spatial availability of free hydroxyl groups preferably shielded by methyl groups. This arrangement gives lignin modified according to the technology described above a high potential for use in biomedicine in the form of a natural preparation to increase the resistance of the organism against negative environmental influences and oxidative stress.

One of the typical causes of increased organism load and oxidative stress is obesity. Increased prevalence but also the incidence of obesity in the adult population as well as in children, i.e. a condition that is rightly referred to as a global epidemic, is largely due to an unhealthy lifestyle associated with lack of exercise and excessive consumption of an unbalanced, energy-rich, low-fibre diet. Obesity is additionally associated with a number of other metabolic complications, collectively referred to as a metabolic syndrome. A key element of these disorders is an insulin resistance whose development involves a number of mechanisms, including chronic inflammation in adipose tissue, oxidative damage associated with increased formation of oxygen radicals as well as metabolic overloading of organs taking part in regulating glucose metabolism in the body, e.g. in skeletal muscle and liver, due to excessive tissue accumulation of lipid metabolism products (Muoio et al., Nature Rev. 2008). Obesity and metabolic syndrome then lead to the development of other serious illnesses, such as type 2 diabetes mellitus and cardiovascular diseases (Van Gaal, Nature, 2006). The treatment of these "civilization" diseases is very complicated and complex, and as a result very burdensome for any healthcare system. A much better option is timely prevention of development of obesity and associated metabolic disorders, for example by using appropriate dietary supplements that are expected to have beneficial effect on key pathophysiological processes associated with obesity and insulin resistance.

Disclosure of the Invention

The present invention solves the problems of the state of the art by a preparation for oral administration comprising lignin, which may be used as a medicament or as a food supplement to prevent and modify metabolic aspects of obesity and/or overweight. The term lignin as used herein includes lignin and bioactivated lignin, prepared for example by the method disclosed in the Slovak document SK280859. Obesity is a state of organism at which an excessive accumulation of adipose tissue occurs, which has negative consequences for the health of the individual. In humans, we talk about obesity when the value of the so-called body mass index (BMI) is 30.0. Conversely, if the BMI is in the range of 25.0 - 29.9, we call it overweight. The preparation for oral administration containing lignin for the prevention of obesity or for reduction of body fat comprises 10 to 99 % w/w of lignin and 1 to 90 % w/w of additives (fillers, binders and carriers, preferably selected from the group comprising gelatine, cellulose, hydroxypropyl cellulose, carboxymethyl cellulose, methyl cellulose, ethyl cellulose, starch, carrageenan, polyvinylpyrrolidone, lactose, sucrose, mannitol, glucose, talc, bentonite, gelatine, cellulose, starch and/or carrageenan capsule shells, water, ethanol, and mixtures of water and ethanol). More preferably, the preparation for oral administration according to the present invention contains 50 to 90 % w/w of lignin and 10 to 50 % w/w of additives, most preferably 60 to 80 % w/w of lignin and 20 to 40 % w/w of additives. The starting material for preparation of bioactivated lignin is a mixture of substances with high lignin content, which is produced as a side product during the manufacture of pulp, starting by neutralization of extract of substances with high lignin content, heating and precipitation, followed by filtration of lignin, washing with water and drying. Preferably, lignin isolated as described in the patent SK280859 is used in the present invention, wherein a sulphate extract formed during delignification of broad-leaved wood with dry-mass content of 60 % and lignin content 22,6 % is diluted by an equivalent volume of water. Sulphuric acid is added dropwise to the stirred reaction mixture until the final concentration of the sulphuric acid reaches 10 %. The precipitated lignin preparation is then filtered, washed with water and dried.
The next step is then alkaline extraction of lignin (preferably at pH > 9, more preferably with 0.1M sodium hydroxide), followed by filtration, washing with water and drying. Fractionation of the modified lignin and filtration of the insoluble component results in isolation of the soluble lignin fraction. After evaporation of the solvents, drying and balancing, the prepared fraction is then repeatedly extracted under alkaine conditions (preferably at pH > 9, more preferably with 0.1M sodium hydroxide) to form an alkaline solution of lignin which is further processed. The said technological process leads to preparation of a biologically active (bioactivated) lignin product that distinguishes from other commercially available lignins by a unique molecular structure, specifically by presence of polymerized polyphenol building blocks that provide for the optimal spatial availability of free hydroxyl groups preferably shielded by methyl groups.
The subject of the present invention is a preparation for oral administration containing 10 to 99 % w/w of lignin and 1 to 90 % w/w of additives for use in the treatment and/or prevention of obesity and/or obesity-induced metabolic complications in humans suffering from obesity, i.e. individuals with a BMI > 30.0. The obesity-induced metabolic aspects mean abdominal fat accumulation, glucose intolerance, hepatic steatosis.
In one preferred embodiment, the preparation for oral administration according to the present invention comprises bioactivated lignin, preparable by a method, wherein a side product from manufacture of pulp, containing lignin, is neutralized, heated and the precipitated lignin is filtered, washed with water and dried. In the next step, lignin is extracted under alkaline conditions, filtered, washed with water and dried. Sunsequently, the modified lignin from the previous step is fractionated, the insoluble component is filtered off, the soluble lignin fraction is evaporated, and the residue is repeatedly extracted under alkaline conditions, giving rise to an alkaline lignin solution.
In a preferred embodiment, the additives are selected from binders, fillers and carriers, preferably selected from the group comprising gelatine, cellulose, hydroxypropyl cellulose, carboxymethyl cellulose, methyl cellulose, ethyl cellulose, starch, carrageenan, polyvinylpyrrolidone, lactose, sucrose, mannitol, glucose, talc, bentonite, and gelatine, cellulose, starch and/or carrageenan capsule shells, water, ethanol, and mixtures of water and ethanol.

In one embodiment, the subject of the present invention is the preparation for oral administration containing lignin for use in the treatment and/or prevention of metabolic aspects of diet-induced obesity in individuals suffering from obesity. The obesity-induced metabolic aspects mean abdominal fat accumulation, glucose intolerance, hepatic steatosis.

In one embodiment, the subject of the present invention is the preparation containing lignin for use in the prevention and/or reduction of abdominal mesenteric (MES) fat depot in individuals suffering from obesity.

In one embodiment, the subject of the present invention is the preparation containing lignin for use in the prevention of impaired glucose homeostasis and/or treatment of disorders of glucose homeostasis in individuals suffering from obesity.

In another embodiment, the subject of the present invention is the preparation containing lignin for use in prevention and/or treatment of increased fat accumulation in the liver (i.e. hepatic steatosis) in individuals suffering from obesity.

In one embodiment, the subject of the present invention is the preparation containing lignin for use, wherein the use comprises adjustment of absorption of diet in the intestine (for example decrease of fat absorption from a diet) and/or positive influence/adjustment of intestinal microflora in individuals suffering from obesity, resulting, as a consequence, in support and increase of immunity of the individual.

The subject of the present invention is also the use of a preparation containing 10 to 99 % w/w of lignin and 1 to 90 % w/w of additives as a food supplement to reduce overweight and/or to improve the overweight-induced metabolic complications in overweight individuals, i.e. people with a BMI ranging from 25.0 to 29.9. Overweight-induced metabolic complications mean abdominal fat accumulation, glucose intolerance, hepatic steatosis.

In one preferred embodiment, the preparation for oral administration according to the present invention comprises bioactivated lignin, preparable by a method, wherein a side product from manufacture of pulp, containing lignin, is neutralized, heated and the precipitated lignin is filtered, washed with water and dried. In the next step, lignin is extracted under alkaline conditions, filtered, washed with water and dried. Sunsequently, the modified lignin from the previous step is fractionated, the insoluble component is filtered off, the soluble lignin fraction is evaporated, and the residue is repeatedly extracted under alkaline conditions, giving rise to an alkaline lignin solution.

In a preferred embodiment, the additives are selected from binders, fillers and carriers, preferably selected from the group comprising gelatine, cellulose, hydroxypropyl cellulose, carboxymethyl cellulose, methyl cellulose, ethyl cellulose, starch, carrageenan, polyvinylpyrrolidone, lactose, sucrose, mannitol, glucose, talc, bentonite, and gelatine, cellulose, starch and/or carrageenan capsule shells, water, ethanol, and mixtures of water and ethanol.

In one embodiment, the subject of the present invention is the use of the preparation containing lignin as a food supplement to improve metabolic aspects of diet-induced overweight in overweight individuals. Metabolic aspects of diet-induced overweight mean abdominal fat accumulation, glucose intolerance, hepatic steatosis.

In one embodiment, the subject of the present invention is the use of the preparation containing lignin as a food supplement for prevention and/or reduction of the abdominal mesenteric (MES) fat depot in overweight individuals.

In another embodiment, the subject of the present invention is the use of the preparation containing lignin as a food supplement to improve glucose homeostasis in overweight individuals.

In another embodiment, the subject of the present invention is the use of the preparation containing lignin as a food supplement to prevent increased fat accumulation in the liver (i.e. hepatic steatosis) in overweight individuals.

The subject of the present invention is also the use of the preparation containing lignin as a food supplement to adjust absorption of diet in the intestine (for example decrease of fat absorption from a diet) and/or positive influence/adjustment of intestinal microflora in overweight individuals, resulting, as a consequence, in support and increase of immunity of the individual.

Brief Description of Figures

Fig. 1: Determination of cytotoxic effects of L, A, and FL lignins using the plating efficiency method on V79/4 cell line; L lignin (■), A lignin (▲), FL lignin (**x**).

Fig. 2: Determination of cytotoxic effects of SD, L, A, and FL lignins using the trypan blue exclusion method on V79/4 cell line; SD lignin (♦), L lignin (■), A lignin (▲), FL lignin (**x**).

Fig. 3: Determination of cytotoxic effects of SD, LSA, and LRA lignins using the trypan blue exclusion method on V79/4 cell line; SD (♦), LSA (■), LRA batch no. 280713 (▲), LRA batch no. 230713 (x), LRA batch no. 010813 (*).

Fig. 4: Determination of genotoxic effects of SD, L, A, and FL lignins with concentrations of 25, 50, and 100 $\mu$g/ml using the comet assay method on V79/4 cell line (** - p > 0.01).

Fig. 5: Determination of genotoxic effects of SD (A), LSA (B), and LRA (C) lignins using the comet assay method on V79/4 cell line.

Fig. 6: Change in body weight due to experimental diets; STD (○), cHF (●), and lignin (△)

Fig. 7: Weight gain during the dietary intervention period (Week 0 to 8)

Fig. 8: Average consumption of diet (STD (○), cHF (●), and lignin (△)) and energy during the trial

Fig. 9: Course of glycemic curves during i.p. GTT (STD (○), cHF (●), and lignin (△))

Fig. 10: Rate of glucose intolerance measured by the area under the glycemic curve (AUC)

Fig. 11: Body weight (A) and weights of two main fat depots, i.e. gonadal depot (B) and subcutaneous depot in dorso-lumbar area (C)

Fig. 12: Weight of liver tissue

Fig. 13: Plasma levels of triacylglycerols in a satiated state

Fig. 14: Plasma levels of total cholesterol (full height of the column) and HDL cholesterol (lower part of the column) in a satiated state

Fig. 15: Change in body weight due to experimental diets with different lignin content; cHF (●), cHF-L1 (x), cHF-L2 (◊), cHF-L4 (△), cHF-L8 (▲), STD (○)

Fig. 16: Weight gain during the dietary intervention period (Week 0 to 9)

Fig. 17: Cumulative energy consumption during the first 7 weeks of the experiment; cHF (●), cHF-L1 (x), cHF-L2 (◊), cHF-L4 (△), cHF-L8 (▲), STD (○)

Fig. 18: Overall energy consumption during the first 7 weeks of the experiment

Fig. 19: Glucose levels during i.p. GTT; cHF (●), cHF-L1 (x), cHF-L2 (◊), cHF-L4 (△), cHF-L8 (▲), STD (○)

Fig. 20: Blood glucose level (glycaemia) after all-night starvation

Fig. 21: Rate of glucose intolerance measured by the area under the glycemic curve (AUC)

Fig. 22: Weight of mesenteric fat tissue

Fig. 23: Effect of lignin on lipid content in the liver tissue (hepatic steatosis)

Fig. 24: Changes in plasma levels of insulin due to lignin

Fig. 25: Plasma TBARS levels at the end of dietary intervention by lignin

Fig. 26: Histological sections of liver tissue stained with haematoxylin - eosin - dark-coloured plaques show damaged tissue

Fig. 27: Amount of stool in 24 hours

Fig. 28: Amount of food absorbed in 24 hours

Fig. 29: Total amount of lipids in stool in 24 hours

Fig. 30: Change in body weight due to deliberate reduction in the diet ration (cHF-PF; the so-called pair-feeding) as compared to the effect of diets with different lignin content; cHF (●), cHF-PF (▲), cHF-L2 (◊), cHF-L4 (△), STD (○)

Fig. 31: Total weight gain over the duration of the experiment (8 weeks)

Fig. 32: Intake of energy by diet during the experiment (from 1st to 6th week); cHF (●), cHF-L2 (◊), cHF-L4 (△), STD (○)

Fig. 33: Blood glucose level (glycaemia) after overnight fasting

Fig. 34: Rate of glucose intolerance measured by the area under the glycemic curve (AUC)

Fig. 35: Plasma insulin levels after overnight fasting

Fig. 36: Weight of mesenteric fat tissue

Fig. 37: Levels of lipopolysaccharide (LPS) in circulation

Fig. 38: Representative histological preparations of intestinal tissue of mice on cHF-L4 diet with 4 % lignin content and of control mice on cHF diet

Examples

The lignins used were tested for their safety, and their cytotoxicity and genotoxicity were experimentally determined

Materials:

The lignin used was isolated from wood processing waste materials as described in SK 280859.

Characterization 1: *Determination of cytotoxicity of lignin fractions*

The V79/4 cell lines were used to determine cytotoxic and genotoxic properties of lignin fractions under "in vitro" conditions. The V79/4 cells represent the queue of Chinese hamster diploid lung fibroblasts and were obtained from Státní zdravotní ústav (National Institute of Public Health) in Prague, Czech Republic. Cells are cultured in RPMI 1640 medium (with 25mM HEPES and L-glutamin) and supplemented with 10% faetal bovine serum and antibiotics - penicillin (100 U/ml), streptomycin (100 g/ml) and kanamycin (100 g/ml). The cells were incubated in plastic Ru-flasks or in plastic 4-well and 12-well plates in $CO_2$ incubator at 37 °C. Two basic "in vitro" methods are used to determine cytotoxic properties of chemicals: plating efficiency and trypan blue exclusion test.

The plating efficiency technique determines the ability of cells to form clones, where one visible colony grows from one single cell. Percentage of cell adhesion is calculated after staining of the clones with methylene blue (1% solution in distilled water).

In brief, the determination procedure is as follows: one hundred V79/4 cells were seeded per well of a 4-well plate (diameter = 3 cm). Cells were incubated for 3 to 4 hours. The RPMI medium was then removed and cells were incubated for 2 hours in RPMI medium containing lignin in concentrations of 25, 50, 100, 200, and 400 $\mu$g/ml. Control cells were maintained in fresh RPMI medium. After exposure to various lignin concentrations, the cells are washed with phosphate buffer and then cultured for 7 days in fresh complete RPMI medium. Upon completion of cultivation, cell colonies are counted and the experiment is evaluated by plotting a graph of dependence of the percentage of colonies of cells grown on the lignin concentration.

The trypan blue method is a technique inducing cell death, membrane lysis and a significant trypan blue penetration into the cell. The V79/4 cells grow in a single layer in the wells of a 12-well plate. Similarly to plating efficiency, cells are affected by different concentrations of lignin preparations (25, 50, 100, 200, and 400 $\mu$g/ml) in complete RPMI medium. Control cells were maintained in fresh RPMI medium. Number of cells in the control group as well as in groups with added lignin is calculated after trypan blue staining of the cells (0.4 %). The percentage of unstained cells indicates the cell line vitality.

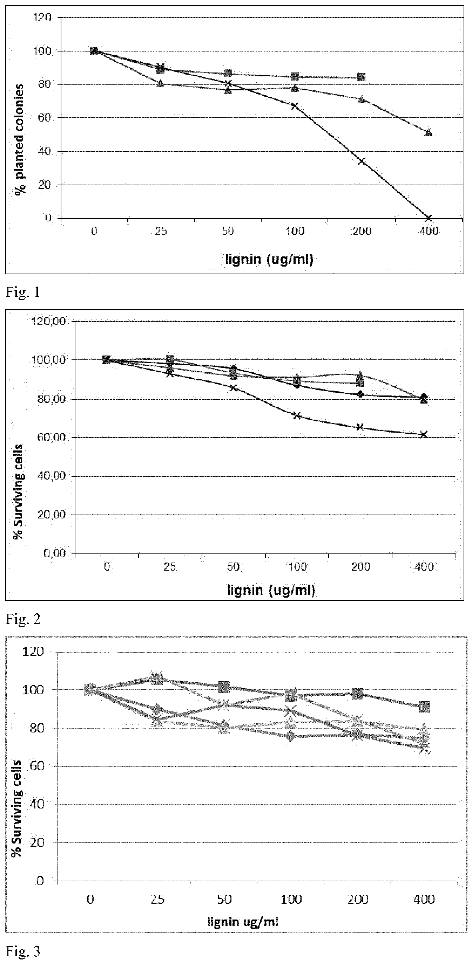

Figures 1 and 2 represent results from experiments that determined non-toxic, possibly low toxic concentrations of lignin samples.

In case of determination of the cloning capability of cells (Fig. 1), lignins isolated from the by-product of pulp production in Štetí were used: L - the lignin fraction after 2nd bioactivation and subsequent lyophilisation; A - the lignin fraction after 2nd bioactivation and without lyophilisation; FL - fractionated lignin. After 2 hours incubation of V79/4 cells with said lignins (25, 50, 100, 200, and 400 $\mu$g/ml), more than 50% colony loss was observed for incubation with FL lignin (at a concentration higher than 100 $\mu$g/ml). A lignin had a slight toxic effect on V79/4 cells from the concentration of 200 $\mu$g/ml and L lignin showed almost no cytotoxic effect.

In case of the trypan blue method, a similar effect was observed for the lignin preparations mentioned above (Fig. 2). The most pronounced cytotoxicity manifested itself again in FL lignin, whose highest concentration caused more than a 70% decrease in cell vitality, while for the remaining lignin products the cytotoxicity did not fall below 80 %. For comparison, a standard control SD lignin preparation - the lignin fraction after 2nd bioactivation and subsequent lyophilisation - Ruzomberok was used in the test. From both determinations, the lowest cytotoxic effect was observed in the case of A and L lignins, which represent lignin fractions obtained after 2nd bioactivation, firstly, without (lignin product A), and secondly, with subsequent lyophilisation of lignin (lignin product L).

Lyophilisation of lignin represents the most time-consuming step in the preparation of lignin.

Because also lignin products after the second bioactivation and without subsequent lyophilisation (lignin product A) exhibit similar noncytotoxic properties as lignin products after the second bioactivation and subsequent lyophilisation (lignin product L), from a time and economic point of view, we would think in the future about elimination of the lyophilisation step from the lignin product preparation process and, consider lignin product A to be our final product. To illustrate and further demonstrate the suitability of use of lignin product A for further "in vitro" and "in vivo" biological tests, Figure 3 shows determinations of noncytotoxic properties of other isolated batches of lignin A type products: LRA - lignin fraction after 2nd bioactivation and without lyophilisation - Ruzomberok; LŠA - lignin fraction after 2nd bioactivation and without lyophilisation - Steti; SD - lignin fraction after 2nd bioactivation and subsequent lyophilisation (standard lignin for comparison) - Ruzomberok.

Characterization 2: *Tests to exclude geuotoxic effects of the lignin product obtained in vitro*

*2.1. Determination of genotoxicity of lignin fractions*

*2.1.1. Genotoxicity - comet assay*

In order to characterize the biological activity of different types of substances of natural and synthetic origin in terms of genotoxicity, various biochemical methods are used. Many of them require DNA molecule labelling by a radioactive isotope, which greatly limits their use in epidemiological studies and clinical practice. In order to avoid the need to work with radioactive material, new methods to detect DNA fractions have been developed. This group also includes the comet assay method.

The comet assay (the single cell gel electrophoresis assay) is a fast and sensitive fluorescence microscopic method for the detection of DNA damage at the level of the individual cell. This technique is based on the ability of DNA strand breaks to migrate in a weak electric field towards the anode, giving rise to a nucleus from which a tail of the "comet" comes out. "Comets" can be visualized by a fluorescence microscope. We recognize two types of comet assays: 1. classic assay - for detection of single-strand breaks and alkali labile sites, and 2. modified assay - for detection of oxidative DNA lesions. In addition, the modified comet assay can be performed using different pH of the electrophoresis solution, pH > 13 and pH = 12.1, to distinguish the DNA lesions of different alkali lability. For the detection of oxidized bases, the function of the repair enzymes formamidopyrimidine-DNA glycosylases is used, which recognizes the oxidized purines, and DNA glycosylases Endo III to distinguish oxidized pyrimidines.

The evaluation of "comets" is based on measurement of DNA percentage parameters in the tail or the "comet" tail length by using special software or visually - by dividing "comets" into individual classes of damage.

The actual determination of the genotoxic effects of lignin preparations took place in the following steps. First, V79/4 cell line was seeded into wells of a 12-well plate and, after reaching the exponential growth phase, the monitored concentrations of lignin (25, 50, and 100 $\mu$g/ml) were added to the individual wells, all incubated for two hours. Upon termination of the lignin effect, the cells were released from the surface of the plates, diluted with an appropriate volume of phosphate buffer to a desired concentration of 1.5 - 2.10$^5$ cells/ml and centrifuged at 1000 rpm. Subsequently, the sediment was resuspended in 75% LMP (low melting point) agarose so that 85 $\mu$l of LMP agarose, which was applied to a pre-prepared microscope slide, contained 2 - 2.5.10$^4$ cells. Smooth slides were used for the experiment, with the surface coated with NMP (normal melting point) agarose. After solidification of the LMP agarose layer with cells at 4 °C and removal of the cover glass, the microscope slides were immersed into lysing solution. Cell lysis was carried out for 60 minutes at 4 °C. At the end of the lysis, the slides were left to drip off and subsequently placed in a horizontal electrophoretic tank where they were allowed to incubate, poured over with electrophoretic buffer (pH > 12.5). Due to the alkaline environment, DNA started to unfold. Upon completion of the unfolding, an electrical current was added to the system and the electrophoresis was performed (25 V, 500 mA). After that the samples were neutralized with neutralizing buffer (0.4M Tris-HCl), 3 times, 5 minutes each. They were stained with 20 $\mu$l of fluorescent dye of ethidium bromide (10 $\mu$g/ml). The preparations thus prepared were evaluated under a fluorescence microscope.

Preparations were evaluated visually. One hundred cells were evaluated from each slide, at least three parallels per sample. Each evaluated cell was ranked in one of the 5 damage classes. The percentage DNA damage is calculated from the sum of damage divided by 400 (all evaluated cells). The sum of damage is obtained by the following conversion:

$$\text{Sum of damage} = \Sigma \ (\text{class of damage x number of cells in the given damage class})$$

The resulting damage may vary from 0 (0 %) (undamaged cells) to 400 (100 %) (all damaged cells).

*2.1.2. Geuotoxic determination of lignins*

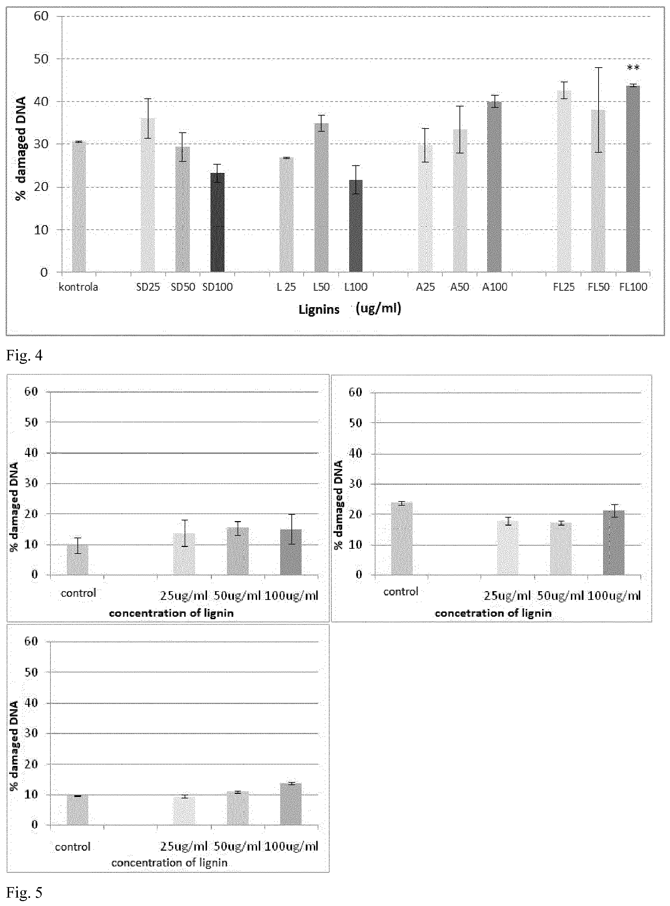

Figure 4 shows the results of the experiments of determination of genotoxic damage of V79/4 cells due to different concentrations (25, 50, and 100 $\mu$g/ml) of lignins, SD - lignin fraction after 2$^{nd}$ bioactivation and subsequent lyophilisation (standard lignin) - Ružomberok; L - lignin fraction after 2$^{nd}$ bioactivation and subsequent lyophilisation - Šteti; A - lignin fraction after 2$^{nd}$ bioactivation and without lyophilisation - Šteti; FL - fractionated lignin - Šteti. Concentrations of 200 and 400 $\mu$g/ml were not used due to a higher cytotoxic effect of lignin observed on V79/4 cells. Controls represent cells, which were instead of a 2-hour effect of lignin incubated for 2 hours in the presence of the solvent itself. As shown in the figure, the genotoxicity of the tested lignins corresponded to cytotoxicity. Again, the highest genotoxic effect was observed with FL lignin, after the incubation of which there was a significant (p > 0.01) increase in the levels of DNA breaks of V79/4 cells in concentration of 100ug/ml in comparison with control. In the case of SD, L, and A lignins, no statistically proven induction of DNA breaks was detected.

As found in the case of cytotoxic determination, even in the case of determination of the genotoxic properties of isolated lignin preparations, the lignin products after second bioactivation and without subsequent lyophilisation (lignin product A) exhibit similar non-genotoxic properties as lignin products after second bioactivation with subsequent lyophilisation (lignin product L). From a time and economic point of view, it would therefore be possible in the future to consider omitting the lyophilisation step from the process for preparation of lignin products and use the lignin product A as the final product.

To illustrate and further demonstrate the suitability of using the lignin product A for further "in vitro" and "in vivo" biological tests, Figure 5 shows the determination of the non-genotoxic properties of other isolated batches of type A lignin products: LRA - lignin fraction after 2$^{nd}$ bioactivation and without lyophilisation - Ružomberok; LSA - lignin fraction after 2$^{nd}$ bioactivation and without lyophilisation - Šteti; SD - lignin fraction after 2$^{nd}$ bioactivation and subsequent lyophilisation (standard lignin for comparison) - Ružomberok.

Example 1: *Tests to eliminate toxic effects of lignin preparation in vivo - Study 1*

*Materials and methods*

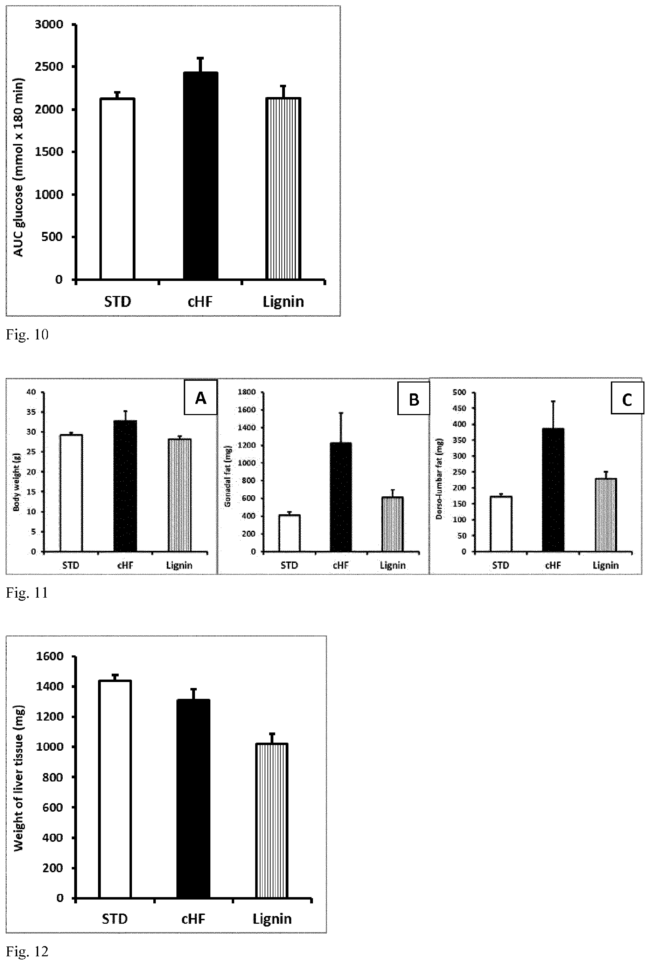

The experiment was performed on male laboratory mice of the inbred C57BL/6J strain. Mice of this strain are highly responsive to high-fat feeding in terms of obesity development, and are thus well suited for experiments aimed at analysing potential influence of various treatments and interventions on impaired metabolism in the context of diet-induced obesity. The disadvantage of this model is, however, quite uneven weight gains in individual mice fed a high-fat diet. Thus, the mice were kept under standard conditions (22 $\pm$ 2 °C, light/dark regime 12h/12h) with *ad libitum* access to a standard low-fat diet (STD) and drinking water. At the age of 4 months, the mice (average weight ~ 27.7 $\pm$ 0.3 g) were divided into three dietary groups (*n* = 8), so that the initial average weight of animals in different subgroups did not differ. Some mice were then fed *ad libitum* experimental high-fat diets (for a detailed description see Table 1 and Figure 10), wherein the control group of mice was fed the high-fat diet based on corn oil (cHF; energy content in lipids ~ 32 %), while the other group of mice was fed the cHF diet supplemented with lignin (cHF-L).

*1. 1. Basic characteristics of the experimental diets*

STD, control low-fat diet: The main macronutrients in this standard diet are complex carbohydrates, namely plant starch. The diet also contains the required amounts of proteins, minerals and fibre, while the total amount of lipids in this diet is relatively low (~3 %) and is predominantly made up of soybean oil with a higher content of essential linoleic acid (C18:3n-3). By its composition the diet is approaching to what wild mice normally eat in nature and is thus a suitable type of "maintenance" diet in adult mice. cHF, control high-fat diet: The main source of lipids in this obesogenic diet is corn oil, which is predominantly made up of omega-6 polyunsaturated fatty acids, primarily linoleic acid (C18:2n-6; ~50 %). This type of fatty acids is associated with the synthesis of proinflammatory lipid molecules in the body, and their presence in the diet promotes the development of obesity and the growth of adipose tissue. Chronic inflammation of adipose tissue in obesity is one of the factors that contribute to the development of obesity-related metabolic disorders (insulin resistance, dyslipidemia, impaired glucose tolerance, etc.). cHF-L, high-fat diet with lignin supplementation: In this diet, the 4 % (by weight) of the original cHF diet is substituted so as to proportionally reduce the content of all other ingredients in the diet. As a result, there was no significant decline in the energy value of this experimental diet. Composition of individual experimental diets is shown in Table 1.

Table 1

|  | STD | cHF | Lignin (cHF-L) |
|---|---|---|---|
| Carbohydrates | 41 % | 24 % | 23 % |
| Fat | 3.3 % | 35 % | 33 % |
| Proteins | 19 % | 20.5 % | 19.5 % |
| Dietary fiber | 5 % | 5 % | 5 % |
| Mineral component | 6.5 % | 6.1 % | 6.1 % |
| Lignin | - | - | 4 % |
| Total energy | 16.3 MJ/kg | 21.4 MJ/kg | 20.97 MJ/kg |

*1.2 Scheme of the experiment*

After assignment of mice to different experimental diets (STD, cHF, Lignin - see above) at the age of 4 months, the animals were reared in cages, two mice per cage, with *ad libitum* access to drinking water and relevant experimental diet. In the first week, mice in cHF-L group were fed only the cHF diet (i.e. lignin-free) due to adaptation to the high-fat feeding conditions and only from the second week, lignin was added to the diet (at 4 %, i.e. 4 g/100 g of diet). In the case of mice in the cHF group, the appropriate diet was fed from the very beginning of the experiment, while mice in STD group continued eating the standard low-fat maintenance diet. The weight of mice and consumption of the respective diets were monitored once a week. The experiment was run for eight weeks and at Week 7 the intraperitoneal glucose tolerance test was carried out. At the termination of the experiment at Week 8, all mice were killed by cervical dislocation under general anaesthesia using diethyl ether. At autopsy, blood from the jugular vein was collected to isolate plasma (by centrifugation at 5,000 g, 10 min, 4 °C), and the relevant organs (i.e. subcutaneous and abdominal adipose tissue, liver, and brown fat) were dissected, weighted and frozen at -80 °C for subsequent biochemical and histological analyses.

*1.3 Intraperitoneal glucose tolerance test (i.p. GTT)*

This test is analogous to the oral glucose tolerance test that is commonly used in clinical practice in patients suspected to have disorders of glucose homeostasis (i.e. impaired glucose tolerance and type 2 diabetes mellitus). In this case, a bolus of glucose is administered intraperitoneally to mice after overnight fasting, which leads to an increase in blood glucose (glycaemia). Increase in glycaemia stimulates the secretion of insulin, which then allows entry of glucose into tissues (muscles, liver, adipose tissue, etc.), which results in subsequent decrease in glycaemia. Changes in glycaemia during the tolerance test (at time 0 and then 15, 30, 60, 120, and 180 min after administration of glucose) are monitored using a glucometer. The result of the measurement is the so-called glycaemic curve, where the course of the curve and the area below it (AUC; area under the glucose curve) provide information on the extent of impairment of glucose homeostasis of the organism. Higher maximum values of glycaemia and/or their slower decrease during the test (i.e. resulting in a higher AUC) indicate the impaired glucose tolerance of the body, which might result from the reduced sensitivity of the body to insulin or its reduced secretion. Both defects are involved in the development of type 2 diabetes mellitus.

*1.4 Results of the Study 1*

1.4.1. *Changes in body weight and food intake*

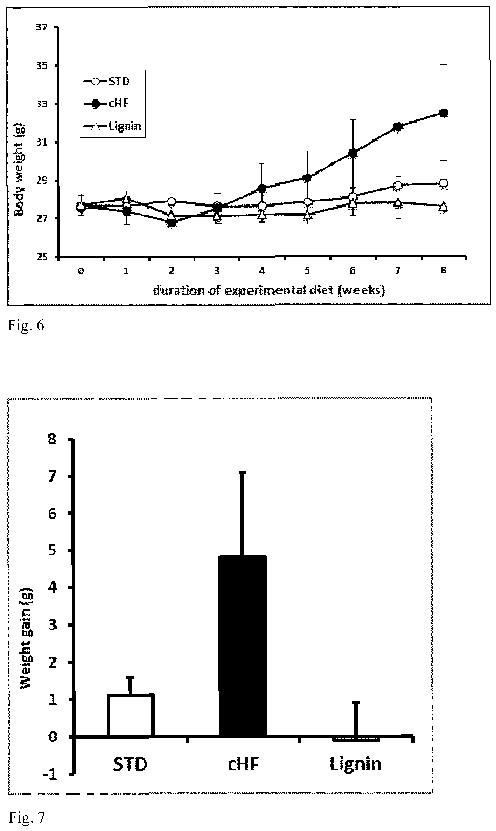

As mentioned above, the obesogenic effect of cHF diet in the control group of mice was relatively heterogeneous, although clearly visible compared to STD group on low-fat maintenance diet (Figs. 6 and 7). On the contrary, the diet containing lignin tended to decrease the body weight (Fig. 6) as well as the overall weight gain ($p = 0.66$ for cHF vs. Lignin; Fig. 7), being comparable in this respect to the effects of STD diet.

Similar conclusions can also be made by comparing the weight gains achieved over the study period (Fig. 7). Despite the considerably heterogeneous weight gains in the groups fed high-fat diet (i.e. cHF and Lignin), the differences between the groups are clearly indicated and e.g. the development of obesity in mice fed the diet containing lignin has been completely suppressed.

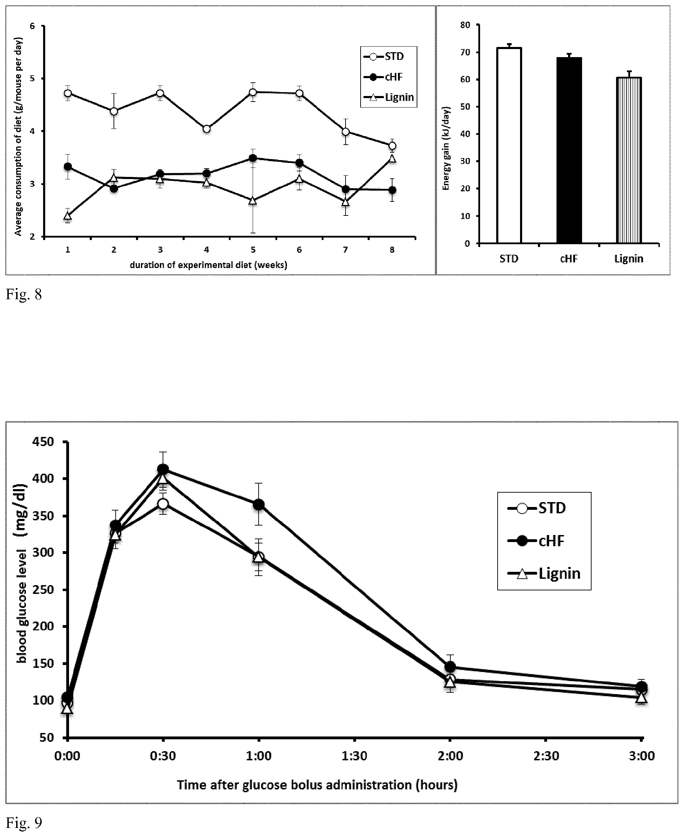

An important aspect in assessing the effect of lignin on body weight gain was its potential impact on energy intake through food (Fig. 8). Average consumption of diet as calculated per mouse and day was relatively stable in individual groups during the experiment (left panel in Fig. 8), where mice in STD group consumed more food than in cHF and cHF-L (i.e. lignin) groups. However, due to significantly lower energy content of STD diet, the average energy consumption in STD and cHF mice was similar (right panel in Fig. 8). In contrast, in the group with lignin (cHF-L), the average energy consumption was lower than in STD group ($p = 0.015$), while in comparison with animals on cHF diet, this decrease was not significant ($p = 0.08$; right panel in Fig. 8).

Given the relatively large variations in the values of the parameters monitored in the individual groups, the indicated reduction in the weight gain in mice fed the diet with 4 % lignin could correlate with the lower energy intake in this intervention group.

1.4.2. *Analysis of glucose homeostasis using i.p. GTT*

During the Week 7 of the experiment, *in vivo* analysis of glucose tolerance using i.p. GTT was carried out in individual mice in order to detect potential effects of lignin on glucose metabolism. As can be seen from Fig. 9, blood glucose levels during the test were lower in STD mice fed the low-fat control diet, and a decrease in glycaemia towards to end of the test is steeper when compared to cHF group that was fed the obesogenic high-fat diet. This is in line with the assumption that glucose tolerance will be better in metabolically healthy mice from STD group than in obese cHF mice. The course of the glycaemic curve observed in mice fed the high-fat diet with lignin ("Lignin"), especially in the phase when glycaemia starts to drop 30 minutes after glucose administration, was similar to the curve observed in STD mice (Fig. 9). This suggests a possible improvement in glucose tolerance due to the lignin supplementation in comparison with animals in the cHF group. However, the level of glucose tolerance characterized by AUC (see above) did not differ significantly between these groups (Fig. 10).

1.4.3. *Changes in the weight of body fat and liver*

At the end of Week 8, the experiment was terminated by killing the animals under general anaesthesia using diethyl ether and collecting blood/plasma and tissues for subsequent analyses. Despite relatively small differences in overall weight gain, significant differences were found in the weight of abdominal (i.e. gonadal; GON) and subcutaneous (i.e. dorso-lumbar; D-L) adipose tissue in mice on STD and cHF diet (Fig. 11). The amount of gonadal and D-L fat was ~3 times (p = 0.035) and ~2 times (p = 0.028) higher, respectively, in mice fed cHF diet in comparison with the animals on low-fat STD diet (Fig. 11). Taking into account the effect of high-fat diet with lignin ("Lignin") on the amount of body fat, only the tendency to decrease the weight of GON (~2x; $p$ = 0.107) and D-L (~1.7x; $p$ = 0.102) fat depots was observed similarly as in case of the effect on weight gain.

The liver plays a key role in the whole-body metabolism of lipids and glucose, and its function in obesity is significantly impaired. One of the accompanying disorders in obesity is the pathological accumulation of lipids in the liver, which is associated with hepatic insulin resistance.

Although the weights of the liver in mice fed the STD and cHF diets did not differ (Fig. 12), the weight of liver in mice with lignin supplementation was significantly reduced ($p$ = 0.011) as compared to animals either on the cHF or STD diet (Fig. 12). These results indicated a potential beneficial effect of lignin on the accumulation of lipids in the liver, which was investigated in another study.

### 1.4.4. *Changes in plasma levels of lipid metabolites*

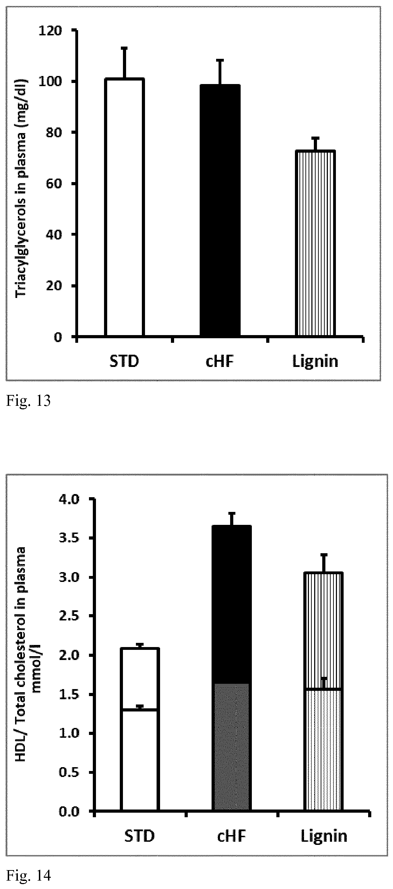

The levels of triacylglycerols (Fig. 13), total cholesterol and HDL-cholesterol (Fig. 14) were analysed in plasma isolated from blood collected during killing in the fed state. While plasma levels of triacylglycerols did not differ significantly (Fig. 13), total cholesterol levels (and also HDL-cholesterol - marked as the bottom part of the individual columns in the graph) were higher in mice on cHF diet in comparison with animals on STD diet (Fig. 14). Lignin supplementation decreased plasma levels of triacylglycerols ($p$ = 0,030; Fig. 13) as compared to control mice fed the cHF diet; in case of total cholesterol, the effect of lignin was on the limit of significance ($p$ = 0.055; Fig. 14).

### 1.5. *Summary of the Study 1*

Study 1 has been performed in order to verify the applicability of this experimental approach for further studies aimed at determining the maximum tolerable concentrations of lignin supplemented in a high-fat diet, especially in terms of its metabolic effects analysed in the context of diet-induced obesity.

Summary of the Study 1:

(i) supplementation of the high-fat diet with lignin at the concentration of 4 % (weight/weight) preserves the dough-like consistency of the experimental diet, which is needed to form daily rations of the diet and which allows accurate measurements of food intake;
(ii) energy intake was slightly reduced in mice consuming the high-fat diet supplemented with lignin (4% dose), and thus for the purpose of the main study it will be useful to analyse the metabolic, antiinflammatory and antioxidant effects of lignin supplemented in concentrations even lower than 4 % (e.g. 1% and 2%) to minimize the potential effect on energy intake;
(iii) due to the considerable variability in measured parameters within the groups of mice fed high-fat diets (i.e. cHF and Lignin), it will be more appropriate to use mice of the C57BL/6N strain, which, unlike the mice of the C57BL/6J strain, respond to high-fat feeding with a relatively homogeneous weight gain;
(iv) plasma levels of some lipid metabolites (e.g. TAG) was not affected by high-fat feeding as compared to mice on low-fat STD diet - this was possibly caused by the fact that animals were killed in the fed state, which represents a situation in which plasma lipid levels are acutely affected by the intake of diets markedly differing in macronutrient composition. Therefore, in the next experiments, the autopsy will be performed either in mice ~6 hours after the removal of their respective dietsor in mice following overnight fasting.

Example 2: *Tests to eliminate toxic effects of lignin preparation in vivo - Study 2*

*Animas*

The experiment was performed on male laboratory mice of the inbred C57BL/6N strain (Charles River Laboratories, Sulzfeld, Germany). Mice at the age of 11 weeks were housed and adapted to standard conditions of the animal room (22 $\pm$ 2 °C, light/dark regime 12h/12h) with *ad libitum* access to STD diet and drinking water. One week later the mice were randomly assigned to different experimental groups ($n$ = 8) so that the average body weight of animals did not differ between the groups.

*Basic characteristics of the experimental diets*

The effects of the following experimental diets were analysed:

1) STD, standard low-fat diet (see the description and detailed composition above);
2) cHF, control high-fat diet (see above);
3) cHF-L1; cHF diet, where 1 % (weight/weight) was substituted with lignin;
4) cHF-L2; cHF diet, where 2 % (weight/weight) was substituted with lignin;
5) cHF-L4; cHF diet, where 4 % (weight/weight) was substituted with lignin (see also the description of the Study 1 above);
6) cHF-L8; cHF diet, where 8 % (weight/weight) was substituted with lignin.

Lignin-supplemented diets were prepared using the same procedure as in the case of the 4% lignin-supplemented diet in the Study 1, i.e. lignin supplementation was based on a proportional substitution of all components contained in the cHF diet, reflecting the percentage of lignin to be supplemented in the cHF diet (i.e. 1, 2, 4 and 8 % - see above).
The choice of 1 and 2 % lignin concentrations was based on the results of the Study 1, in which 4% lignin was used; on the other hand, the highest 8 % lignin concentration was already expected to cause a significant reduction in food intake and/or other side effects.

*Experimental design*

After assigning mice to different experimental diets at the age of 12 weeks (see above), the animals were housed individually in the cages, with *ad libitum* access to drinking water and respective experimental diets. Experimental design was similar as in case of the Study 1, however the mice in this study were placed on experimental diets without the initial 1 week adaptation to the cHF diet. The experiment lasted for 9 weeks, while the analysis of glucose homeostasis by i.p. GTT was performed at Week 8 of the experiment. Body weight and food consumption were monitored once a week, with an accurate measurement of food consumption due to individual housing of mice. At the end of experiment, mice were killed by cervical dislocation under diethyl ether anaesthesia, blood was collected from the cervical veins to isolate plasma while relevant organs/tissues, i.e. GON and D-L (see Study 1 for description), as well as abdominal mesenteric adipose tissue (MES), brown fat and the liver) were dissected, weighted and flash-frozen in liquid nitrogen for the subsequent storage at -80 °C.

*Results of the Study 2*

2.1. *Changes in body weight and food intake*

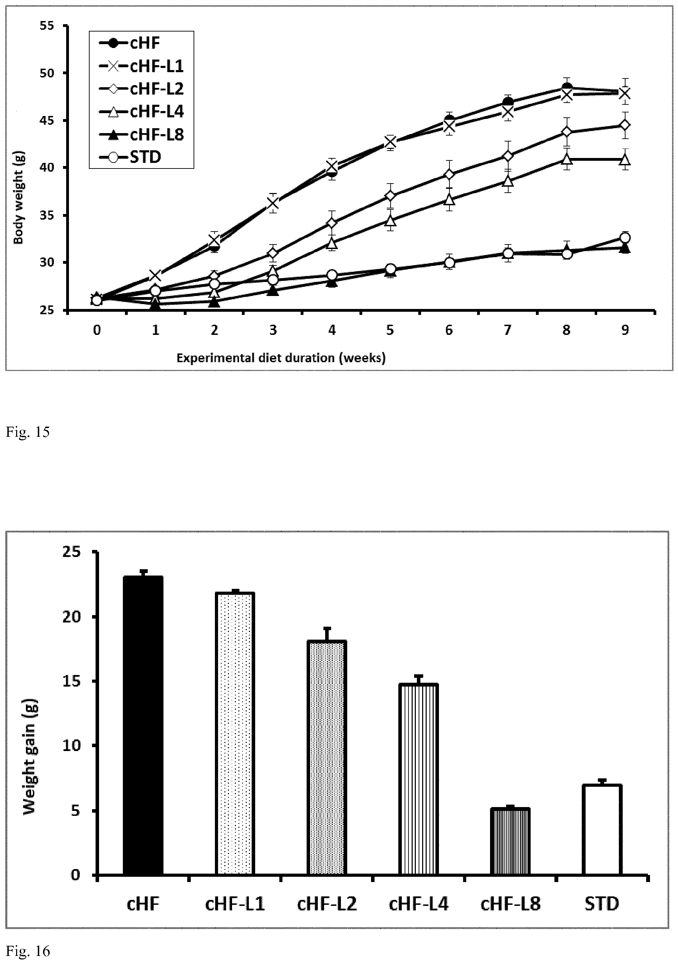

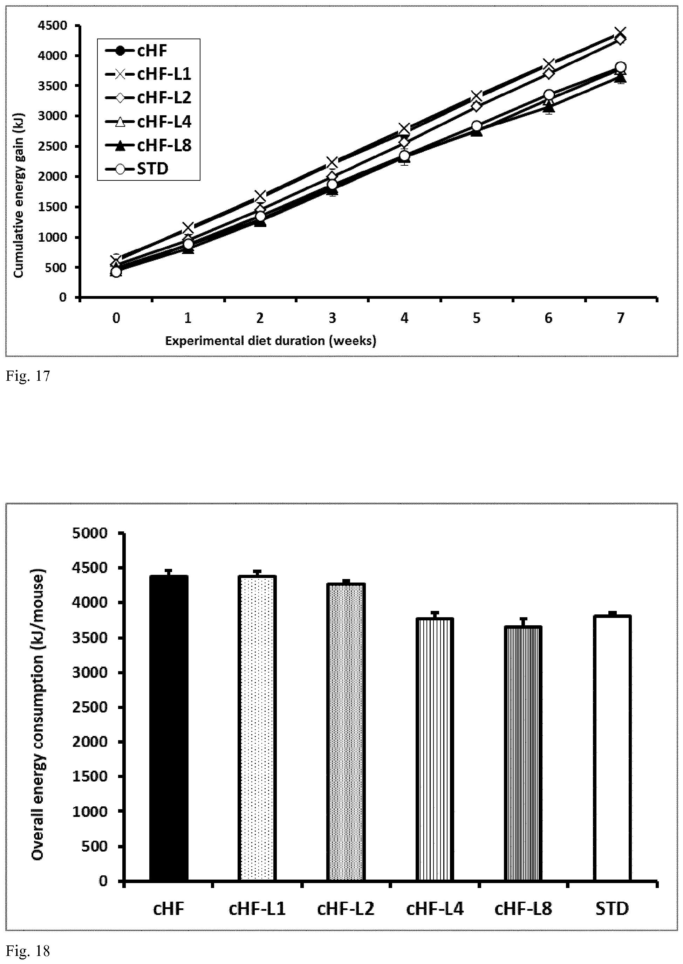

In comparison with the low-fat STD diet, the obesogenic effect of cHF diet in the control group of mice was clearly visible and relatively homogenous (Fig. 15). On the contrary, lignin supplementation decreased body weight of mice (Fig. 15) depending on the dose in which it was supplemented in the cHF diet. While the lowest concentration of lignin (i.e. 1 %; cHF-L1 diet) did not differ from the control cHF diet in terms of its effect on body weight gain, the highest concentration (i.e. 8 %; cHF-L8 diet) largely prevented weight gain in mice, which was similar to that observed in mice fed the low-fat STD diet. The other tested concentrations of lignin (i.e. 1 % and 2 %, corresponding to the cHF-L2 and cHF-L4 diet, respectively) had similar effects on body weight of mice (Fig. 15). These data were in line with the calculated weight gain achieved from the beginning of the experiment (Week 0) until the end of study at Week 9 (Fig. 16). Thus, in this study, the body weight gain in cHF mice was ~22 g, while in the pilot study it was only ~5 g. Therefore, in this experiment it was possible to "unmask" the antiobesogenic effects of lignin and accurately titrate its optimal doses.
Together with the measurement of body weight, the energy intake through diet and its impact on the body weight gain during the first 7 weeks of the experiment was also assessed. The cumulative energy intake of individual mice within the given subgroups was quite homogenous (Fig. 17), and did not differ between the cHF, cHF-L1 and cHF-L2 group, however it was still higher than in the STD, cHF-L4 and cHF-L8 group (Fig. 17 and Fig. 18). Compared to control animals fed the cHF diet, the total energy intake in mice fed the cHF-L1 or cHF-L2 diet was not different, whereas in mice consuming the highest doses of supplemented lignin (i.e. in the cHF-L4 and cHF-L8 group) it was significantly reduced (Fig. 18; $p < 0.001$ for both diets).

2.2. *Glucose homeostasis*

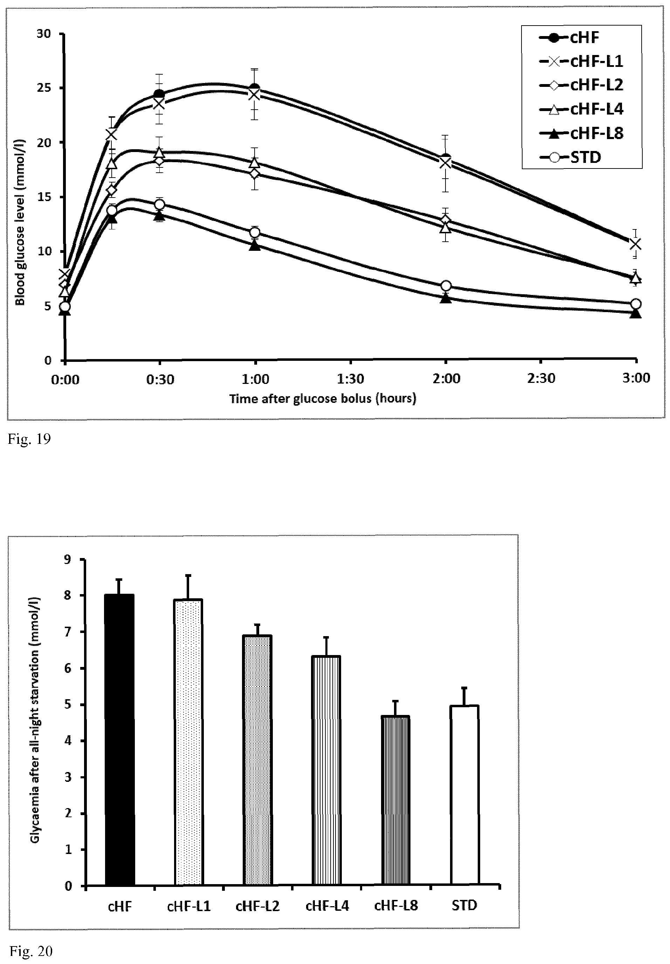

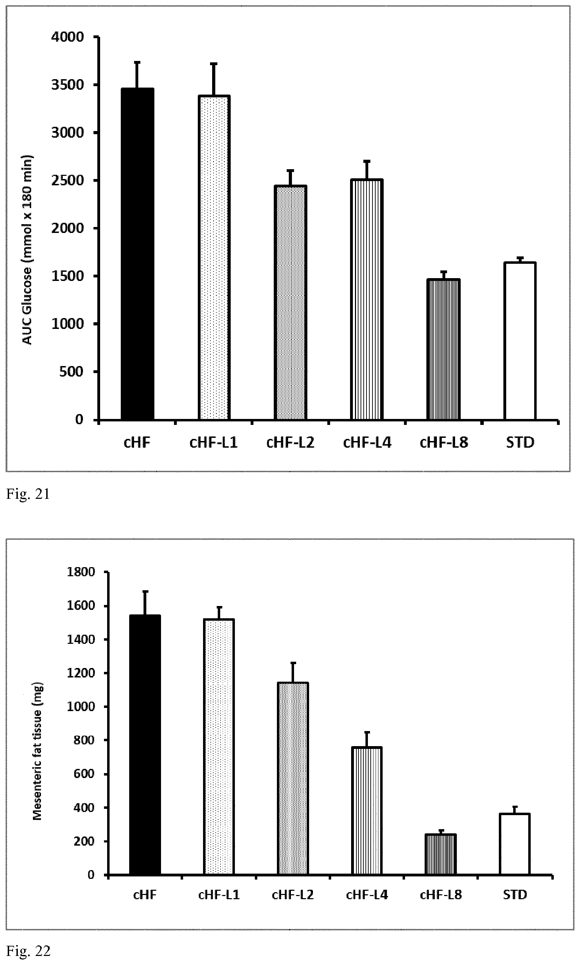

During the Week 8, the state of glucose homeostasis was analysed using i.p. GTT (Fig. 19). As expected, fasting blood glucose levels (Fig. 20; $p < 0.001$) as well as glycaemia during the GTT (Fig. 19) and the corresponding AUC (Fig. 21; $p < 0.0001$) were significantly increased in cHF mice as compared to STD-fed controls, which indicated a significant impairment of glucose tolerance due to feeding the obesogenic cHF diet. Conversely, lignin supplementation of cHF diet led to a significant improvement in all of the above parameters in a dose-dependent manner (Figs. 19-21). It is noteworthy that feeding the cHF-L2 diet (i.e. lignin administered at a dose of 2 %) resulted in similar beneficial effects on glucose homeostasis as in case of animals fed the cHF-L4 diet ($p < 0.05$ vs. cHF for fasting blood glucose and AUC; Fig. 20 and 21), although the cHF-L2 unlike the cHF-L4 diet did not significantly affect energy intake in mice (see Fig. 17).

*2.3. Changes in the weight of body fat depots and the liver, the effect of lignin on hepatic steatosis*

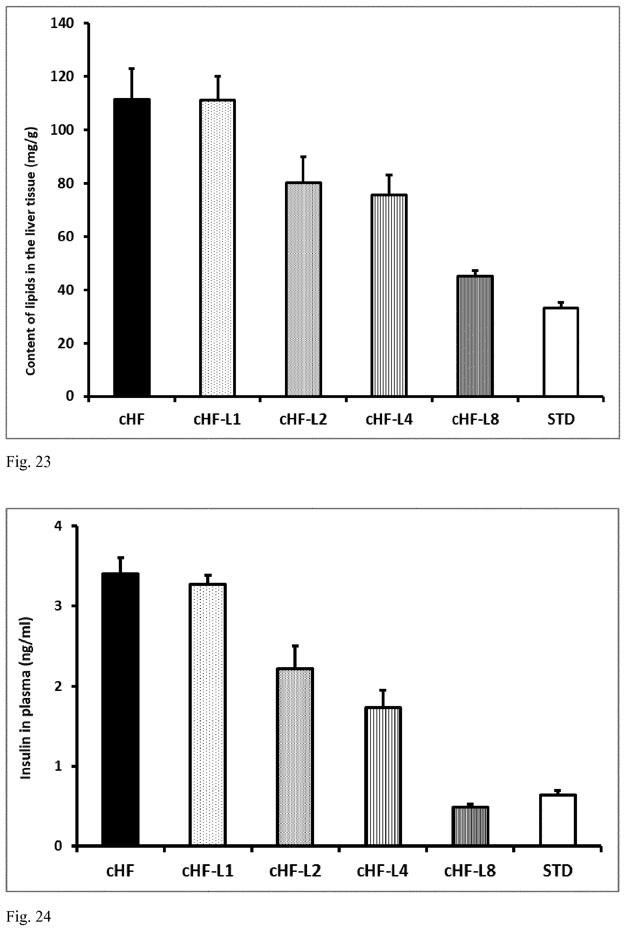

As described above, supplementation of the cHF diet with lignin led to a reduction in body weight gain depending on the lignin dose (see Fig. 16). At the end of study at week 9, mice were killed and the weight of individual fat depots including D-L, GON, and MES) was measured (Fig. 22). The weight of individual fat depots in mice fed the cHF diet was about 4 times higher as compared to STD-fed lean controls. Dietary supplementation with lignin led to a reduction in adiposity, although this reduction was dependent on the type of fat depot and the lignin dose. While the weight of D-L and GON was not significantly affected by lignin, except the highest dose (not shown), the weight of MES (Fig. 22) decreased due to lignin in a dose-dependent manner. The preferential effect of lignin on this type of abdominal adipose tissue, which is deposited around the intestines and drained by the portal vein directly into the liver, could be of great importance in terms of the effect on metabolic complications in obesity. The reduction of the liver weight due to lignin was also dose-dependent (not shown). Changes in the liver weight could be partly explained by the effect of lignin on hepatic lipid content, i.e. hepatic steatosis (Fig. 23), which is promoted by obesity and insulin resistance, and represents an independent risk factor for cardiovascular disease. Hepatic lipid accumulation in cHF mice was ~3.4 times higher when compared to controls fed the low-fat STD diet; on the other hand, lignin in a dose-dependent manner decreased the level of hepatic steatosis, while the effects of the cHF-L2 and cHF-L4diet were comparable (Fig. 23).

*2.4. Changes in plasma lipid metabolite and insulin levels*

Plasma levels of lipid metabolites, including triacylglycerols, free fatty acids and total cholesterol were analysed using biochemical methods, while immunoreactive insulin in plasma was detected by the RIA method (Fig. 24). As with the pilot study, lignin supplementation reduced the levels of triacylglycerols (not shown), however this was observed only in mice fed the highest dose of lignin (i.e. cHF-L8 diet; $p < 0.02$ vs. cHF), while the effect on plasma cholesterol levels in mice fed the cHF-L8 diet was at the limit of significance ($p = 0.07$; not shown). The levels of free fatty acids were not significantly affected by lignin (not shown). While plasma insulin levels were markedly increased in obese mice fed the cHF diet as compared to lean animals on STD diet (Fig. 24), thus indicating the induction of insulin resistance in obese mice. Lignin supplementation decreased plasma insulin levels in a dose-dependent manner (Fig. 24; cHF-L2, $p < 0.01$; cHF-L4, $p < 0.0001$; cHF-L8, $p < 0.0001$ vs. cHF), wherein the lowest dose of lignin (i.e. 1 %; cHF-L1 diet) had no effect. Of note, the highest dose of lignin (i.e. 8%; cHF-L8 diet) reduced plasma levels of insulin to those of lean control mice fed the STD diet.

*2.5. Changes in plasma levels of tissue damage markers*

In order to monitor potential harmful effects of lignin supplementation on the organism, markers of tissue damage were analysed in plasma of mice fed different experimental diets. Thus, plasma levels of alanine aminotransferase (ALT) and aspartate aminotransferase (AST; BioLa tests from Erba Lachema, Brno, Czech Republic), whose elevated concentration in circulation may indicate damage to the cytoplasmic membrane of hepatocytes, were determined. Compared to obese controls fed the cHF diet, plasma levels of AST in mice with lignin supplementation were not different (not shown), while the ALT levels were reduced by lignin in a dose-dependent manner, however with a significant difference (vs. cHF mice) observed only in case of the highest lignin dose, i.e. cHF-L8 diet (cHF, $0.63 \pm 0.07$ vs. cHF-L8, $0.38 \pm 0.05$ µcat/1; $p = 0.023$). In addition, the enzymatic activity of lactate dehydrogenase (LDH) in plasma (measured by the kit from Erba Lachema) was characterized by a high variability within the individual subgroups of mice, and it was not affected by lignin supplementation (not shown). Overall, it can be concluded that lignin supplementation does not exert toxic effects on tissues, at least on the basis of the concentration of tissue damage markers in circulation.

*2.6. Oxidative stress markers in plasma and in tissues*

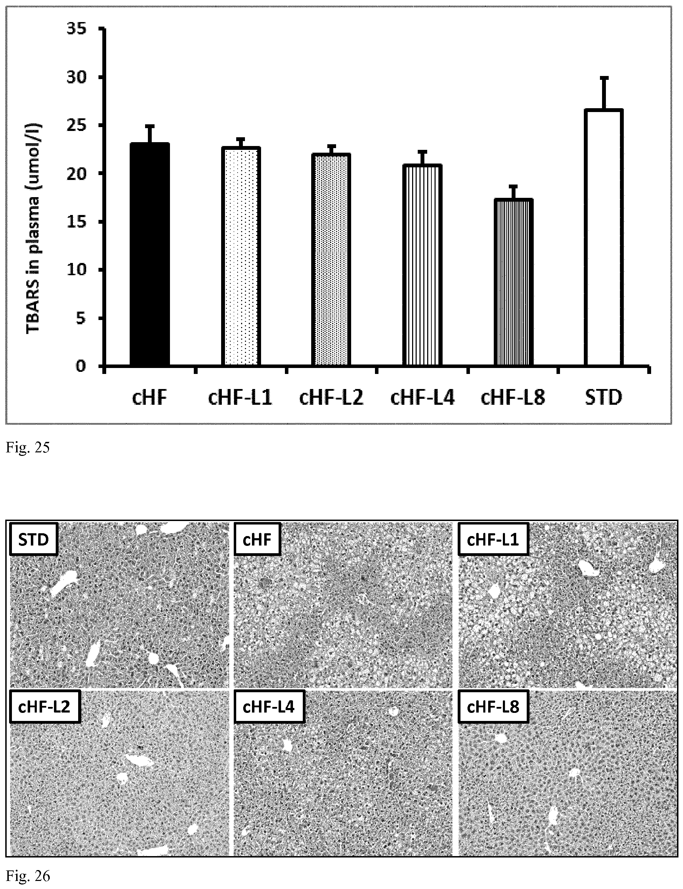

Measurements of oxidative stress markers in plasma (TBARS) and in adipose tissue and liver (TBARS and catalase) were performed using the OxiSelect™ TBARS Assay Kit and OxiSelect™ Catalase Activity Assay Kit (Cell Biolabs, San Diego, USA). In general, TBARS, which reflect the level of lipid peroxidation (the detection is based on the formation of the adduct of malondialdehyde with thiobarbituric acid), did not differ between the lean mice fed the STD diet and obese controls on the cHF diet, however they were reduced in plasma of mice with lignin supplementation, depending on the dose (Fig. 25); however significant effect was observed only in the group of cHF-L8 mice (cHF, 23 $\pm$ 1 vs. cHF-L8, 18 $\pm$ 1 $\mu$mol/l; $p$ = 0.016). In adipose tissue, the concentration of TBARS was not altered due to lignin supplementation, while in the liver TBARS were higher by ~35 % in mice fed the highest dose of lignin (cHF, 25 $\pm$ 2 vs. cHF-L8, 34 $\pm$ 1 nmol/g of tissue; $p$ < 0.01). Enzyme activity of catalase in adipose tissue and liver did not differ between the obese control cHF mice and the mice supplemented with lignin, except for the highest concentration of lignin which slightly increased the catalase activity (by less 10 %) in the liver (not shown). Of note, the catalase activity in adipose tissue and the liver of lean mice fed the STD diet was higher than in obese cHF-fed controls.

*2.7. Histological analysis of liver tissue*

Histological analysis was performed in samples of liver fixed in formaldehyde and embedded in paraffin. The analysis of histological sections of liver tissue stained with haematoxylin-eosin confirmed the results of biochemical quantification of tissue lipids (i.e. differences in the level of hepatic steatosis; see above) and further indicated that lignin supplementation in a dose of 1 to 8 % did not cause pathological changes in the tissue (Fig. 26).

2.8. *The amount of stool and quantification of lipids in the stool*

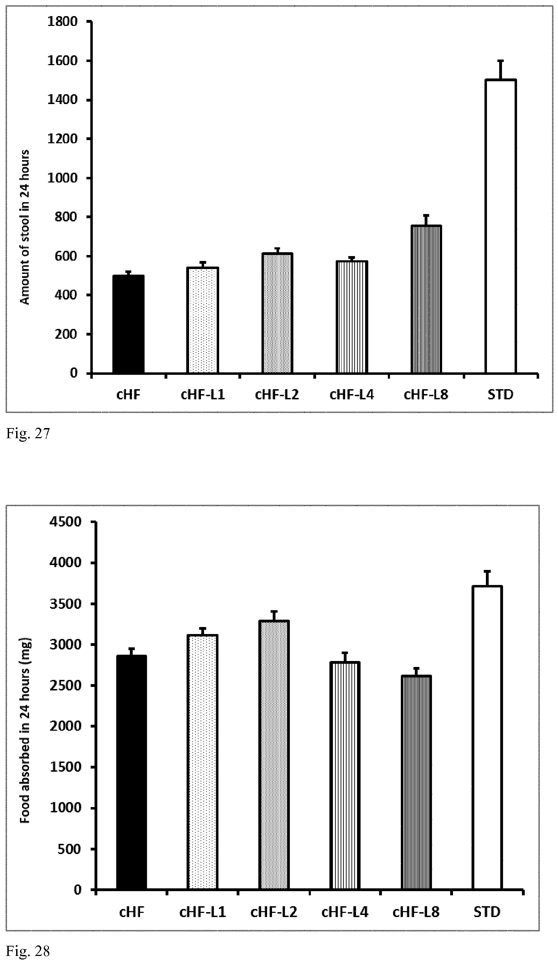

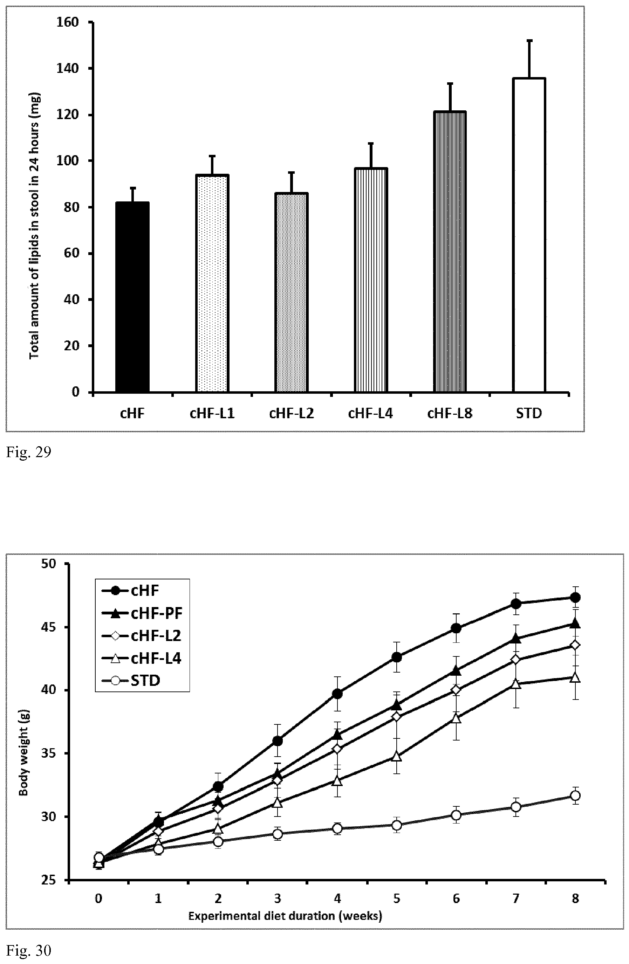

Lignin supplementation could induce changes in the metabolism and absorption of nutrients in the gastrointestinal tract. The total content of lipids in stool samples collected from individual mice over a 24-hour period was analysed by gravimetry. During the same period of time, food consumption was measured to estimate the amount of absorbed food. Thus, lignin supplementation tended to increase dose-dependently (starting at a dose of 2%) the amount of stool produced during a 24-hour period as compared to obese cHF controls (Fig. 27), with the cHF-L8 group being significantly different compared to all other groups. Similarly in mice fed the low-fat STD diet the amount of stool was ~3 times higher than in obese cHF-fed mice (Fig. 27), while the amount of absorbed food was also increased in the STD group (Fig. 28). On the contrary, in mice supplemented with lignin at a dose of 8 % (i.e. cHF-L8 diet) the amount of absorbed food was lower as compared to cHF-fed controls, as well as in comparison with animals fed diets containing 1 % and 2 % lignin (i.e. the cHF-L1 and cHF-L2 diet). Interestingly, the amount of absorbed food in the cHF-L2 group was even higher than in cHF controls (Fig. 28). Regarding the content of lipids in the stool, it was increased in mice supplemented with 8% lignin (i.e. cHF-L8 diet) compared to both the control cHF group and the cHF-L2 group, however it did not differ from the lean controls fed the STD diet (Fig. 29).

*2.9. Summary of the Study 2*

This study showed that the body weight gain in mice of the C57BL/6N strain was higher and more homogeneous within the experimental groups when compared to mice of the C57BL/6J strain analyzed in the Study 1. Very good quality and consistency of high-fat diets supplemented with lignin enabled testing the effects of lignin supplemented in a relatively wide range of concentrations. Thus, the results of this Study 2 suggested that:

(i) with the exception of 1% lignin dose (i.e. cHF-L1 diet), all the higher doses of lignin (i.e. 2, 4, and 8 %) led to measurable effects on energy homeostasis and metabolism in mice fed a high-fat obesogenic diet, with dose-dependent effects observed in most cases;
(ii) with the exception of 1 and 2 % lignin doses, energy intake in mice fed the high-fat diets supplemented with 4 and 8 % lignin content has been reduced, so it is not possible to determine exactly to what extent the beneficial metabolic effects of these lignin doses possibly depend on the reduced energy intake;
(iii) the highest dose of lignin (i.e. 8 %) in mice fed the cHF-L8 diet reduced the body weight gain even below that of lean controls fed STD diet, likely due to a reduced energy intake; however, given the metabolic improvements and the absence of elevated plasma levels of tissue damage markers observed in cHF-L8 mice, it can be assumed that this highest dose tested is nontoxic and safe;
(iv) the reduction of body weight gain in mice fed either the cHF-L2, cHF-L4, or cHF-L8 diet was not reflected in the proportional loss of all the fat depots analysed, but instead it induced a preferential loss of MES (i.e. mesenteric adipose tissue around the intestines), which could be important in terms of influence on the pathophysiology of metabolic complications in obesity;
(v) the effect of lignin supplementation on plasma markers of lipid metabolism in obese mice fed a high-fat diet was relatively weak, whereas improvements in parameters of glucose homeostasis such as fasting blood glucose, glucose tolerance, and plasma insulin were pronounced and affected by lignin in a dose-dependent manner;
(vi) lignin supplemented in the cHF diet seems to represent a safe way of influencing metabolism through diet, since no toxic manifestations of its administration were detected in the tissues (based on plasma markers of tissue damage including AST, ALT, and LDH, as well as histological analysis of liver tissue);

(vii) chronic supplementation of lignin in the cHF diet decreased systemic levels of TBARS (a marker of lipid peroxidation) in a dose-dependent manner;

(viii) as compared to mice fed the cHF diet alone, lignin supplementation dose-dependently increased the volume of stool, wherein the 4 and 8 % doses of lignin also reduced the volume of diet absorbed in the intestines, while the 1 and 2 % doses had the opposite effect;

(ix) lignin supplementation tended to increase lipid content in the stool, but only the highest 8 % dose exerted a significant effect.

Example 3: *Comparing the effects of lignin supplementation and calorie restriction on energy balance and metabolism in vivo - Study 3*

The aim of the next study was to verify whether the metabolic effects of lignin supplementation could depend on a reduced calorie intake observed in case of higher lignin doses, e.g. doses of 4 and 8 %. Thus, an experiment analysing the effects of the cHF diet administered to mice in the amount corresponding to the level of energy intake observed in mice supplemented with lignin at a dose of 4 % (i.e. the cHF-L4 diet) was performed. Besides the groups of mice fed the cHF and cHF-L4 diet, and mice subjected to "pair-feeding" (cHF-PF; see above), also mice fed the STD and cHF-L2 diet were used in this study. Glucose tolerance test was carried out one week before the end of experiment, i.e. after 7 weeks on experimental diets. The experiment was terminated after the end of the eighth week of dietary interventions.

3.1. *Methodology*

3.1.1. *Animals*

Male mice of the inbred C57BL/6N strain were used for the experiment (Charles River Laboratories, Sulzfeld, Germany). Mice at the age of 10 weeks were adapted to standard conditions of the animal room (22 $\pm$ 2 °C, light/dark regime 12h/12h) with *ad libitum* access to a standard low-fat diet (Ssniff R/M-H diet; Ssniff Spezialdieten GmbH, Soest, Germany) and drinking water. At the age of ~12 weeks, animals were randomly assigned to experimental groups (*n* = 8), so that the average body weight of mice did not differ between the groups. Mice were fed the experimental diets (see below) for a period of 8 weeks.

3.1.2. *Experimental diets*

Groups of mice were fed the following experimental diets:

- STD, standard low-fat diet;

  - cHF, control high-fat diet based on corn oil, in which the majority of fatty acids are the omega-6 fatty acids (mainly linoleic acid, 18:2n-6);

- cHF-L2; cHF diet, where 2 % (weight/weight) was substituted with lignin;
- cHF-L4; cHF diet, where 4 % (weight/weight) was substituted with lignin;
- cHF-PF; cHF diet, the amount of which was reduced to reflect the energy intake in the group of mice fed the cHF-L4 diet (i.e. "pair-feeding").

For details on the STD, cHF and cHF-L (i.e. lignin-supplemented) diets, including the procedure of their preparation, see Materials and methods in Study 1 above.

All the main procedures in this study are described in the description of Studies 1 and 2 above.

3.2. *Results of the Study 3*

3.2.1. *Changes in body weight and food intake*

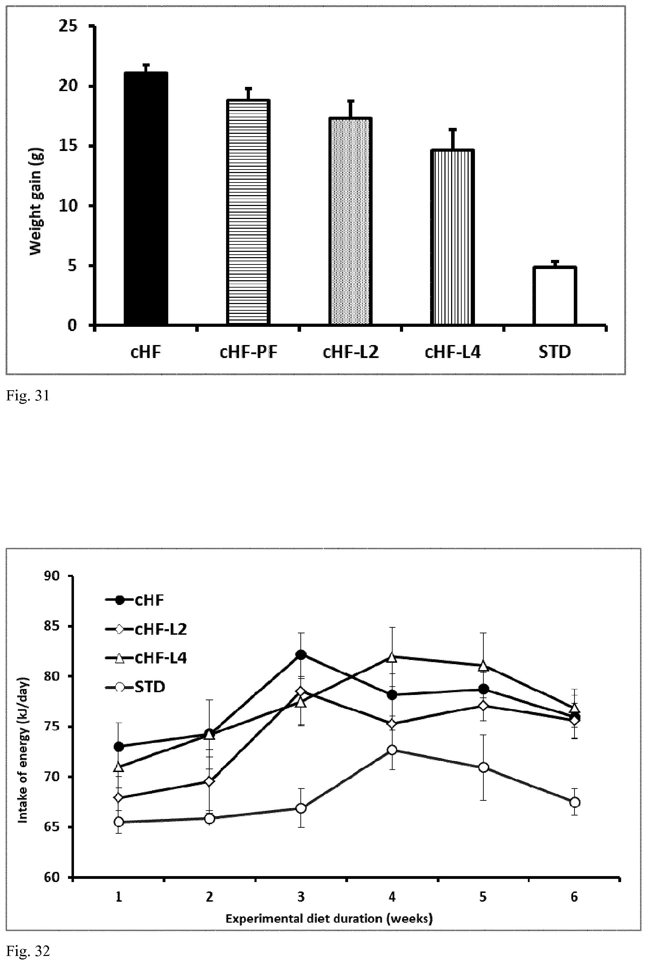

Feeding obesogenic cHF diet for 8 weeks resulted in a marked increase in body weight compared to animals on low-fat STD diet (Fig. 30); the body weight gain of mice fed the cHF diet was ~21 g, which was roughly 4 times more than in case of mice fed STD diet (Fig. 31). Feeding diets supplemented with lignin (i.e. cHF-L2 and cHF-L4) resulted in a significant reduction of weight gain as compared to obese mice fed the cHF diet (Fig. 31). In addition, in case of mice fed the cHF-L4 diet (i.e. lignin dose of 4 %), this reduction was more pronounced ($p < 0.05$) as compared to mice from the cHF-PF group, which consumed the same amount of energy. The energy intake in mice fed the cHF diet did not differ significantly from energy intake of mice fed diets containing lignin (Fig. 32).

*3.2.2. Glucose homeostasis*

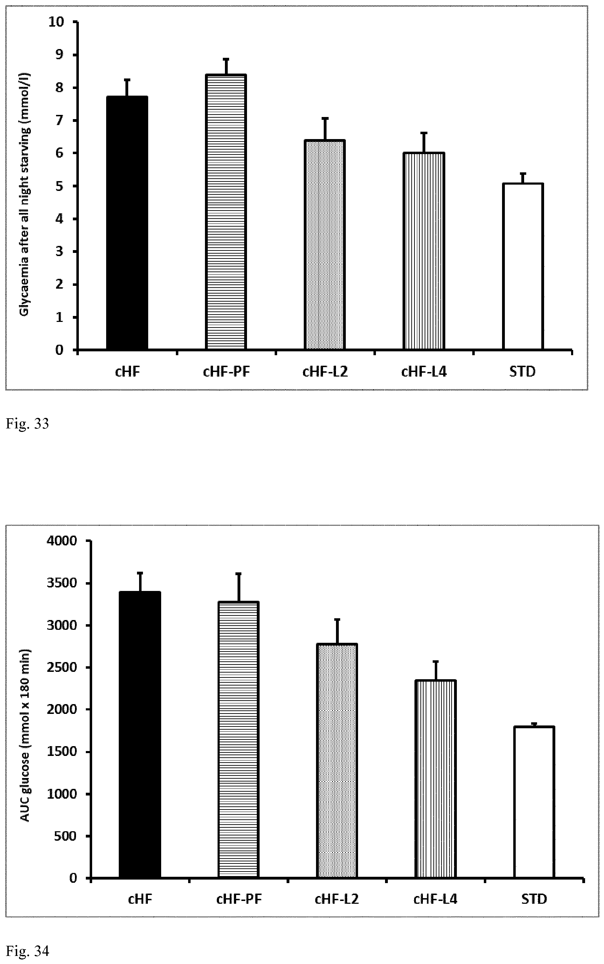

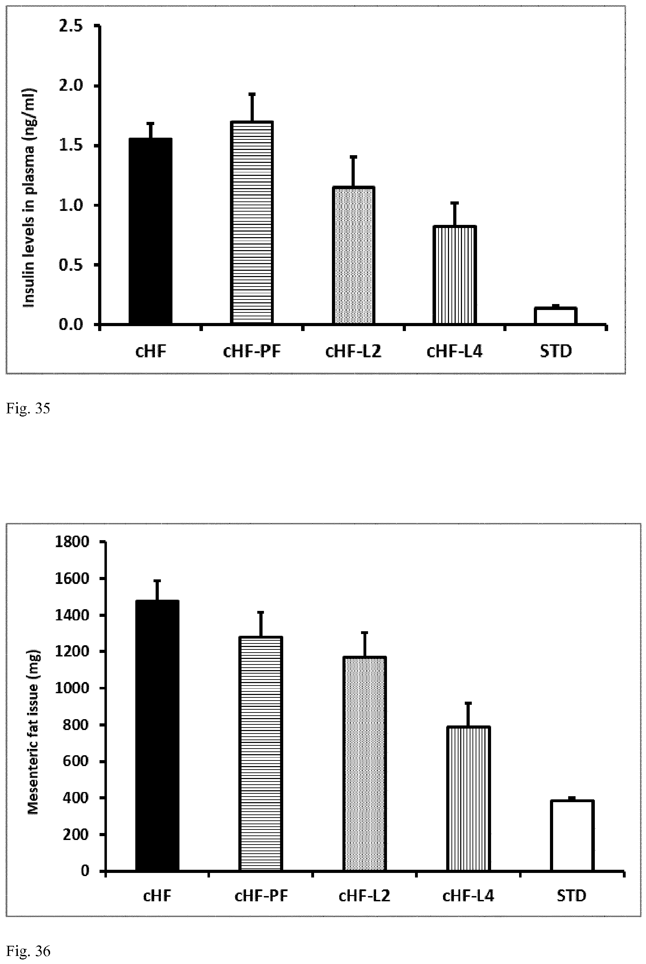

At the beginning of Week 8 of the experiment, glucose homeostasis was analysed by i.p. GTT. Blood glucose levels after overnight fasting were elevated by ~50 % in obese mice fed the cHF diet as compared to lean STD-fed controls (Fig. 33), while fasting blood glucose in mice fed the cHF-L4 diet tended to be decreased ($p = 0.057$). Mice fed the lignin-supplemented diets (i.e. cHF-L2 and cHF-L4 diet) also exhibited lower blood glucose levels as compared to pair-fed mice (i.e. the cHF-PF group; Fig. 33). The level of glucose intolerance expressed as AUC (Fig. 34; see also Materials and methods in Study 1 above for details) was the highest in obese cHF-fed mice, as their AUC was increased by ~ 90 % as compared to lean STD-fed controls. In contrast, the supplementation of cHF diet with lignin led to a significant improvement in glucose tolerance (i.e. revealed as lower AUC). Importantly, lignin supplementation at 4 % (i.e. cHF-L4 diet) also improved glucose tolerance when compared to pair-fed mice in the cHF-PF group. The levels of plasma insulin in the fasted state (Fig. 35), i.e. a surrogate marker of insulin resistance, were consistent with the results of glucose tolerance testing. Thus, feeding mice the obesogenic cHF diet resulted in a dramatic increase in plasma insulin levels as compared to lean mice fed the low-fat STD diet, indicating the induction of insulin resistance; on the contrary, feeding the cHF-L4 diet containing 4 % lignin resulted in a significant reduction of insulin levels as compared to both mice fed the cHF diet and pair-fed mice in the cHF-PF group (Fig. 35).

*3.2.3. Changes in the amount of body fat*

After 8 weeks of dietary interventions mice were killed and adipose tissue from the GON, D-L and MES fat depot was quantitatively dissected. As compared to control animals fed the STD diet, the weight of fat depots in mice fed the obesogenic cHF diet was increased ~4 times (see the weight of MES in Fig. 36), thus corresponding well to the differences in body weight gain. The supplementation of cHF diet with lignin, especially at a 4% dose (i.e. cHF-L4 diet), tended to decrease the weight of GON and D-L fat depot (not shown), however lignin preferentially affected the weight of MES (Fig. 36), which showed a reduction in both groups supplemented with lignin, i.e. in mice fed the cHF-L2 diet ($p = 0.058$) and cHF-L4 diet depending on the lignin dose. The effect of the latter diet was even stronger than that of calorie restriction (i.e. in the cHF-PF group), thus suggesting that lignin could act independently, at least partially, of calorie restriction.

*3.2.4. Plasma levels of lipid metabolites and LPS*

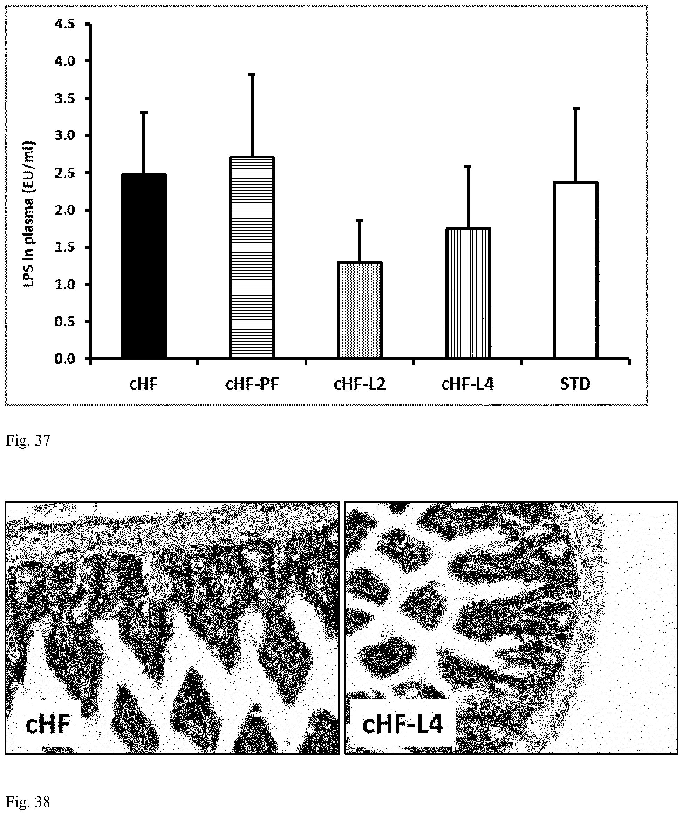

Plasma samples, isolated from blood collected in the morning hours (i.e. in the post-absorptive state) during the dissection of mice, was used to measure the levels of lipid metabolites. However, in line with our previous results (see above), lignin at a dose of 2 or 4 % did not significantly affect plasma levels of any of the analysed lipid metabolites, i.e. triacylglycerols, free fatty acids and total cholesterol (not shown). Obesity is associated with a greater proportion of G-bacteria in the intestines, thus leading to a production of lipopolysaccharides (LPS), which could leak into circulation due to increased intestinal permeability and potentiate the development of insulin resistance. Since lignin supplementation could affect the intestinal microflora, plasma levels of lipopolysaccharides (LPS; also known as endotoxins) were analysed (Fig. 37). However, they were not different between the obese and lean controls fed the cHF and STD diet, respectively, and were not affected by lignin supplementation (Fig. 37).

*3.3. Summary of the Study 3*

The results of the experiments performed within the Study 3 showed that:

(i) lignin at a dose of 2 and 4 % has significant anti-obesogenic effects, whereas the effect of 4 % lignin on body weight gain was even stronger than that of calorie restriction in mice from the cHF-PF group, which consumed the same amount of energy;
(ii) similarly to previous studies, lignin supplementation at a dose of 2 and 4 % improved glucose tolerance; in addition, 4% dose improved glucose tolerance and reduced fasting blood glucose and plasma insulin levels more than calorie restriction in the cHF-PF group;
(iii) lignin preferentially reduced the weight of abdominal MES fat depot without significantly altering gene expression of enzymes involved in fatty acid metabolism (not shown); as before, the effect of 4 % lignin was significantly stronger than that of calorie restriction (i.e. in the cHF-PF group).

Example 4: *Histological analysis of the intestinal tissue from mice supplemented with lignin - Study 4*

This study focused on histological analysis of intestinal tissue removed from obese controls fed the cHF diet and mice fed the cHF-L4 diet containing 4 % lignin. The aim of this experiment was to rule out the possibility that lignin supplementation induces pathological changes in the intestinal tissue. Animals, diets, and experimental design were analogous to previous studies.

After 9 weeks of dietary interventions mice were killed by cervical dislocation and samples from the terminal part of the *ileum* were collected, fixed in 4 % paraformaldehyde, embedded in paraffin, and tissue sections stained with haematoxylin-eosin. The quality and integrity of the intestinal villi and musculature, as well as signs of tissue inflammation or pathological adhesions were evaluated. Histological analysis (Fig. 38) did not detect any gross pathological changes or inflammatory processes in the intestinal tissue from mice fed the lignin-supplemented cHF-L4 diet as compared to obese cHF-fed controls.

**Claims**

1. A preparation for oral administration containing 10 to 99 % w/w of lignin and 1 to 90 % w/w of additives for use in the treatment and/or prevention of obesity and/or obesity-induced metabolic complications in humans suffering from obesity.

2. The preparation for use according to claim 1, **characterized in that** it comprises bioactivated lignin preparable by a method, wherein a side product from manufacture of pulp, containing lignin, is neutralized, heated and the precipitated lignin is filtered off, washed with water and dried; in the next step, lignin is extracted under alkaline conditions, filtered, washed with water and dried, and, subsequently, the modified lignin from the previous step is fractionated, the insoluble component is filtered off, and the soluble lignin fraction is isolated by evaporation and repeated extraction of the residue under alkaline conditions, giving rise to an alkaline lignin solution.

3. The preparation for use according to claim 1 or 2, **characterized in that** the additives are selected from binders, fillers and carriers, preferably selected from the group comprising gelatine, cellulose, hydroxypropyl cellulose, carboxymethyl cellulose, methyl cellulose, ethyl cellulose, starch, carrageenan, polyvinylpyrrolidone, lactose, sucrose, mannitol, glucose, talc, bentonite, and gelatine, cellulose, starch and/or carrageenan capsule shells, water, ethanol, and mixtures of water and ethanol.

4. The preparation for use according to any one of claims 1 to 3 for use in the treatment and/or prevention of metabolic aspects of diet-induced obesity in individuals suffering from obesity.

5. The preparation for use according to any one of claims 1 to 3 for use in the prevention and/or reduction of abdominal mesenteric fat depot in individuals suffering from obesity.

6. The preparation for use according to any one of claims 1 to 3 for use in the prevention of impaired glucose homeostasis and/or treatment of disorders of glucose homeostasis in individuals suffering from obesity.

7. The preparation for use according to any one of claims 1 to 3 for use in prevention and/or treatment of hepatic steatosis in individuals suffering from obesity.

8. The preparation for use according to any one of claims 1 to 3, wherein the use comprises adjustment of absorption of diet and/or positive adjustment of intestinal microflora in individuals suffering from obesity.

9. Use of a preparation containing 10 to 99 % w/w of lignin and 1 to 90 % w/w of additives, preferably selected from binders, fillers and carriers, as a food supplement to reduce overweight and/or to improve the overweight-induced metabolic complications in overweight individuals.

10. Use according to claim 9, **characterized in that** the preparation for oral administration comprises bioactivated lignin preparable by a method, wherein a side product from manufacture of pulp, containing lignin, is neutralized, heated and the precipitated lignin is filtered off, washed with water and dried; in the next step, lignin is extracted under alkaline conditions, filtered, washed with water and dried, and, sunsequently, the modified lignin from the previous step is fractionated, the insoluble component is filtered off, and the soluble lignin fraction is isolated by evaporation and repeated extraction of the residue under alkaline conditions, giving rise to an alkaline lignin solution.

11. Use according to claim 9 or 10 to improve metabolic aspects of diet-induced overweight in overweight individuals.

12. Use according to claim 9 or 10 for reduction of the abdominal mesenteric fat depot in overweight individuals.

13. Use according to claim 9 or 10 to improve glucose homeostasis in overweight individuals.

14. Use according to claim 9 or 10 to prevent increased fat accumulation in the liver in overweight individuals.

15. Use according to claim 9 or 10 to adjust absorption of diet and/or positive adjustment of intestinal microflora in overweight individuals.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

EP 3 354 269 A1

Fig. 25

Fig. 26

29

Fig. 27

Fig. 28

Fig. 29

Fig. 30

Fig. 31

Fig. 32

Fig. 33

Fig. 34

Fig. 35

Fig. 36

Fig. 37

Fig. 38

Europäisches
Patentamt

European
Patent Office

Office européen
des brevets

**EUROPEAN SEARCH REPORT**

Application Number

EP 18 15 3496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/075539 A2 (SATIOGEN PHARMACEUTICALS INC [US]; GEDULIN BRONISLAVA [US]; GREENE HOW) 23 June 2011 (2011-06-23) | 1-4,6, 9-11,13 | INV. A61K31/765 A61K8/86 A61K36/00 A23L29/206 A23L33/00 A61P3/06 A61P3/04 A61P3/08 A61P3/10 |
| Y | * claims 1, 3, 4, 5, 18, 22, 27, 38, 47 * | 1-15 | |
| X | SATO SHIN ET AL: "Effect of lignin-derived lignophenols on hepatic lipid metabolism in rats fed a high-fat diet.", ENVIRONMENTAL TOXICOLOGY AND PHARMACOLOGY SEP 2012, vol. 34, no. 2, September 2012 (2012-09), pages 228-234, XP002781525, ISSN: 1872-7077 * the whole document * * page 230, right-hand column, last paragraph * * figure 2; tables 1, 2, 3 * | 1-6,9-13 | |
| X | CA 2 841 735 A1 (TOLBA RASHA [CA]) 6 August 2015 (2015-08-06) * the whole document * | 1,2,4, 9-11 | TECHNICAL FIELDS SEARCHED (IPC) A61K A23L A61P |
| Y | SHIN SATO ET AL: "Lignin-derived lignophenols attenuate oxidative and inflammatory damage to the kidney in streptozotocin-induced diabetic rats", FREE RADICAL RESEARCH, vol. 43, no. 12, 1 January 2009 (2009-01-01), pages 1205-1213, XP55479893, GB ISSN: 1071-5762, DOI: 10.3109/10715760903247264 * table 1 * | 1-15 | |

-/--

The present search report has been drawn up for all claims

| Place of search | Date of completion of the search | Examiner |
|---|---|---|
| Munich | 6 June 2018 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 18 15 3496

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 98/41216 A1 (TAM YUN K [CA]; ZAMAN NUZHAT [CA]; ISHIDA NAOBUMI [CA]) 24 September 1998 (1998-09-24) * claim 1 * | 7 | |
| A | US 3 721 735 A (THIFFAULT C) 20 March 1973 (1973-03-20) * the whole document * | 1-15 | |

TECHNICAL FIELDS
SEARCHED (IPC)

The present search report has been drawn up for all claims

| Place of search | Date of completion of the search | Examiner |
|---|---|---|
| Munich | 6 June 2018 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 18 15 3496

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2018

| Patent document cited in search report | | Publication date | Patent family member(s) | | Publication date |
|---|---|---|---|---|---|
| WO 2011075539 | A2 | 23-06-2011 | GB<br>US<br>WO | 2476384 A<br>2011152204 A1<br>2011075539 A2 | 22-06-2011<br>23-06-2011<br>23-06-2011 |
| CA 2841735 | A1 | 06-08-2015 | NONE | | |
| WO 9841216 | A1 | 24-09-1998 | AU<br>HK<br>JP<br>WO | 2089297 A<br>1027508 A1<br>2001514667 A<br>9841216 A1 | 12-10-1998<br>25-11-2005<br>11-09-2001<br>24-09-1998 |
| US 3721735 | A | 20-03-1973 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SK 28259

- SK 280859

**Non-patent literature cited in the description**

- Lignin structure and morphological distribution in plant cell walls. **HIGUCHI, T.** Lignin biodegradation: Microbiology, Chemistry, and Potential Application. CRC Press, 1980, 1-7
- **GLASSER, W.G.** *Forest Products Journal,* 1981, vol. 31, 24-29

- **KRATZL, K. ; SCHAEFER, W. ; CLAUS, P. ; GRATZ, J. ; SCHILLING, P.** *Monatsh. Chem.,* 1964, vol. 98, 891-904
- **LU FJ ; CHU LH ; GAU RJ.** *Nutr Cancer,* 31 August 1998, vol. 30 (1
- **MUOIO et al.** *Nature Rev.,* 2008
- **VAN GAAL.** *Nature,* 2006